(19) Europäisches Patentamt
European Patent Office
Office européen des brevets

(11) EP 1 480 155 A2

(12) EUROPEAN PATENT APPLICATION

(43) Date of publication:
**24.11.2004 Bulletin 2004/48**

(51) Int Cl.[7]: **G06F 19/00**

(21) Application number: **04252807.5**

(22) Date of filing: **14.05.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **19.05.2003 JP 2003140793**

(71) Applicant: **CANON KABUSHIKI KAISHA**
**Ohta-ku, Tokyo (JP)**

(72) Inventor: **Yoshii, Hiroto,**
**c/o Canon Kabushiki Kaisha**
**Ohta-ku, Tokyo (JP)**

(74) Representative: **Beresford, Keith Denis Lewis**
**BERESFORD & Co.**
**16 High Holborn**
**London WC1V 6BX (GB)**

(54) **Information processing apparatus, information processing method, storage medium and program**

(57) Biological species are determined easily, inexpensively, in a short time, and accurately even if a plurality biological species having base sequences similar to one another exist in a specimen. For achieving such an object, the information processing apparatus according to the present invention is an information processing apparatus processing information about the signal intensity of each probe obtained as a result of making a predetermined specimen undergo a hybridization reaction using a DNA micro-array in which probes being nucleic acid complementary to some of nucleotide sequences of biological species.

FIG. 1

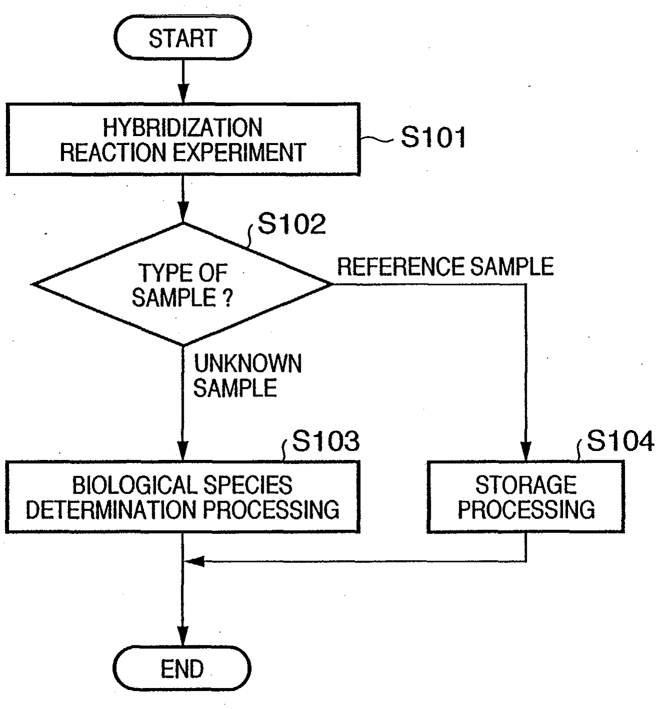


EP 1 480 155 A2

## Description

FIELD OF THE INVENTION

The present invention relates to a nucleotide sequence analysis using so-called a DNA micro array-array, and particularly to a technique for determining the type of microorganism such as bacteria.

BACKGROUND OF THE INVENTION

There has been a technique called a "culture method" as a technique for determining causative bacteria of an infectious disease appearing on a patient. In this method, bacteria contained in blood taken from the patient are cultured in a specified culture medium, and grown bacteria are observed to determine causative bacteria of the infectious disease.

This technique has a disadvantage that several days are required for determination of causative bacteria, and it is thus almost impossible to determine causative bacteria before determining a course of treatment for the patient. That is, it is desired that causative bacteria should be determined before administering a therapeutic drug such as an antibiotic substance to the patient, but if the result of determination must be awaited for several days, the possibility is increased that the condition of the disease of the patient worsens and is past remedy. Therefore, drugs matching a plurality of possible causes must be administered before determination of causative bacteria and as a result, the patient should take a risk of side effects for the efficacy of the drugs.

One method for solving this problem is a method of determining causative bacteria of an infectious disease by a DNA analysis of causative bacteria. In this method, a specified site having a genome in causative bacteria is amplified using, for example, the PCR (polymerase chain reaction) method and the LAMP method, and the nucleotide sequence of the amplified site is read to determine causative bacteria, and according to such a method, it is possible to determine causative bacteria in a short time, and a plurality of causative bacteria can be determined irrespective of the type of causative bacteria.

Furthermore, as another method for solving the above problem, for example, U.S. Patent No. 6040138 discloses a method in which amplified nucleic acid fragments derived from a target organism are made to undergo a hybridization reaction with nucleic acid fragments called probes to determine the amount of the nucleic acid fragments derived from the target organism.

According to the specification (U.S. Patent No. 6040138), use of a densely integrated DNA micro-array allows a large number of probes to be set for one target nucleic acid fragment and as a result, information equivalent to that obtained by reading the sequence of the target nucleic acid fragment can be obtained in a short time.

Of the methods shown in the above conventional techniques, however, the method of determining causative bacteria of an infectious disease by the DNA analysis of causative bacteria has a problem such that a very high technical skill and cost are required for reading the nucleotide sequence. The DNA amplification method such as the PCR method or LAMP method itself can be carried out by a simple procedure, but requires a high purity for amplified nucleic acid fragments in order that the nucleotide sequence can be read. Furthermore, an "apparatus for reading nucleotide sequences" called a sequencer is expensive, and the operation for reading the nucleotide sequence is much more complicated than the DNA amplification by the PCR method and the LAMP method, and is often hard to be performed with a skill of an ordinary inspection technician.

On the other hand, the method by the hybridization reaction (method described in U.S. Patent No. 6040138) requires a lower skill and cost compared to the sequencer, but requires a process for determining a biological species based on the result of the hybridization reaction. For determining the biological species here, a method so called "homology search" is generally used, but such a method has a problem such that it is difficult to distinguish between causative bacteria having similar base sequences. This is because the "homology search" is a method of determining the existence probabilities based on the result of the hybridization reaction, thereby determining the biological species.

Generally, for distinguishing between causative bacteria having similar base sequences in the homology search, it is a major prerequisite that "different probes set for different types of nucleic acid fragments are mutually independent" in the hybridization reaction in the DIVA micro-array. For example, if there are 10 probes matching a gene A, it is a prerequisite that a nucleic acid fragment derived from a gene B does not react with the probes. Only the prerequisite allows existence/nonexistence (existence probability) of causative bacteria to be determined correctly based on the estimated amount of the gene A obtained by averaging the signal intensity (for example fluorescence intensity) obtained as a result of the hybridization reaction of the 10 probes, for example.

However, if nucleic acid fragments are similar to one another even though they are derived from different biological species, the above prerequisite such that probes are mutually independent is not practical and for example, the gene B may undergo the hybridization reaction even with a probe designed for the gene A (such a phenomenon is referred to as "cross hybridization"). Therefore, the above method of "determining the existence probability of a target nucleic acid fragment using a representative value obtained by the operation of averaging over a plurality of probes matching the target nucleic acid fragment" is not practical for determination of causative bacteria in a specimen having a plurality of similar base sequences, and thus has a problem such that determination accuracy obtained by the method is lacking in reliability.

SUMMARY OF THE INVENTION

The present invention has been made in view of the above problems, and its object is to determine biological species easily, inexpensively, for a short time and accurately when a plurality of biological species having base sequences similar to one another exist in a specimen.

For achieving the above object, an information processing apparatus according to the present invention has the following configuration. Specifically,

an information processing apparatus processing information about the signal intensity of each probe obtained by subjecting a predetermined specimen to a hybridization reaction using a DNA micro-array where the each probe is arranged, the each probe nucleic acid complementary to a partial nucleotide sequence of a nucleic acid of biological species, the information processing apparatus comprises:

retaining unit configured to retain first information as information about the signal intensity of the each probe obtained by subjecting any known biological species to the hybridization reaction;
acquiring unit configured to acquire second information as information about the signal intensity of the each probe obtained by subjecting the predetermined specimen to the hybridization reaction;
extracting unit configured to extract information related to a predetermined biological species from the first and second information; and
determining unit configured to determine whether or not the predetermined specimen may comprise the predetermined biological species by making a comparison between information related to the predetermined biological species, extracted from the first information by the extracting unit, and information related to the predetermined biological species from the second information.

According to the present invention, when a plurality of biological species having base sequences similar to one another exist in a specimen, the biological species can be determined easily, inexpensively, in a short time and accurately.

Other features and advantages of the present invention will be apparent from the following description taken in conjunction with the accompanying drawings, in which like reference characters designate the same or similar parts throughout the figures thereof.

BRIEF DESCRIPTION OF THE DRAWINGS

The accompanying drawings, which are incorporated in and constitute a part of the specification, illustrate embodiments of the invention and, together with the description, serve to explain the principles of the invention.

FIG. 1 shows a flow of overall inspection including an information processing method of the present invention;
FIG. 2 is a block diagram showing the configuration of an information processing apparatus for realizing the information processing method (biological species determination method) according to one embodiment of the present invention;
FIG. 3 shows the state of hybridization on a DNA micro-array;
FIG. 4 is a view for explaining an overall experimental procedure of a hybridization reaction experiment using the DNA micro-array;
FIG. 5 is a view for explaining the principle of the DNA micro-array for determining bacteria of an infectious disease;
FIG. 6 is a view for explaining the reason why a plurality of types of base sequences exist in a hybridization solution.
FIG. 7 shows one example of an image representing a fluorescence intensity after a hybridization reaction;
FIGS. 8A and 8B show one example of a vector distribution map and a classification tree for determining the same;
FIG. 9 is a functional block diagram for explaining the process of a biological species determination method according to one embodiment of the present invention;
FIG. 10 shows an example of the DNA micro-array having a plurality of spots of probes of the same type;
FIG. 11 shows one example of a primitive vector filter for use in the information processing method according to one embodiment of the present invention;
FIG. 12 is a flowchart showing a flow of main component analysis processing;
FIG. 13 is a view for explaining s learning phase and a pattern recognition phase;

FIG. 14 is a flowchart showing a flow of classification tree creation processing; and
FIG. 15 is a view for explaining steps of determining a node determination function.

DETAILED DESCRIPTION OF THE PREFERRED EMBODIMENTS

Preferred embodiments of the present invention will now be described in detail in accordance with the accompanying drawings.

FIG. 1 shows a flow of overall inspection processing including an information processing method according to the present invention. As shown in this figure, in inspection, a hybridization reaction experiment with a known specimen is first carried out using a DNA micro-array (step S101), information (scan image) about the signal intensity represented by the fluorescence intensity of the DNA micro-array obtained as a result is stored as the result of the reaction of a reference sample (steps S102 and S104), and then processing for determination of a biological species is carried out based on the result of the reaction of the reference sample for information (scan image) about the fluorescence intensity of the DNA micro-array obtained as a result of a reaction experiment for an unknown sample (steps S102 and S103). Processes of steps S101 and S103 will be described step by step below.

1. Description of hybridization reaction experiment (step S101)

[1-1. Flow of hybridization reaction experiment]

First, an overall experimental procedure of a hybridization reaction experiment using a DNA micro-array will be described using FIG. 4.

A "sample" denoted by reference numeral 401 refers to a specimen such as a liquid or solid that should contain target nucleic acid. For example, if causative bacteria of an infectious disease are determined, samples include all entities considered as containing bacteria such as blood derived from humans and animals such as livestock, body fluids such as expectoration, gastric juice, vaginal secretions and intraoral mucosa, excrements such as urea and feces. Furthermore, media that could cause contamination by bacteria, such as food products suspected of causing food poisoning and contamination, and water in the environment such as potable water and hot spring water, may be used as samples. Further, animals and plants subjected to medical inspection when they are imported or exported are also targeted.

The sample 401 is amplified using a "biochemical amplification" method denoted by reference numeral 402. For example, if causative bacteria of the infectious disease are determined, target nucleic acid is amplified by the PCR method using a primer for PCR reaction designed for 16s rRNA detection, or the PCR-amplified substance is used as an original and further adjusted by a PCR reaction or the like. Furthermore, it may be adjusted by an amplification method such as the LAMP method other than the PCR method.

The amplified sample or original sample 401 is labeled by various kinds of labeling methods for detection of signal intensity (label incorporation 403). In the present invention, the signal intensity is the signal intensity capable of being detected and measured as appropriate by appropriate means, and includes fluorescence, radioactivity and chemiluminescence, but the fluorescence intensity is preferable. For the labeling substance for this purpose, a fluorescent substance such as Cy3, Cy5 or Rodamin is preferably used. Furthermore, in biochemical amplification processing (402), a labeling molecule may be incorporated.

Nucleic acid with a labeling molecule added thereto is used to carry out a hybridization reaction (405) with a DNA micro-array 404 (details are described later). For example, if causative bacteria of the infectious disease are determined, probes specific to bacteria fixed on a substrate are used as the DNA micro-array 404. The probe of each type of bacteria is designed from a genome site coated with, for example, 16s rRNA so that it is highly specific for the bacteria, and can be expected to have a hybridization sensitivity free from variations "where possible" with each probe base sequence. A carrier (substrate) on which the DNA micro-array 404 is fixed can be a plane substrate such as a glass substrate, plastic substrate or silicon wafer. Furthermore, a bumpy three-dimensional structure, s spherical structure such as a bead, a rod-shaped structure, a braid-shaped structure, a string-shaped structure and the like may be used.

The substrate is usually surface-treated so that the probe DNA can be fixed. Particularly, a substrate having a functional group on the surface so that a chemical reaction can proceed is preferable in terms of reproducibility because the probe can be stably bound in the process of the hybridization reaction.

Furthermore, for fixation, for example, a combination of a maleimide group and a thiol (-SH) group is used. Specifically, the thiol (-SH) group is bound to the end of the nucleic acid probe, and a solid phase surface is treated so that it has the maleimide group, whereby the thiol group supplied to the solid phase surface reacts with the maleimide group on the solid phase surface to fix the nucleic acid probe.

For introduction of the maleimide group, an amino silane coupling agent is first made to react with a glass substrate. Then, the maleimide group is introduced by the reaction between the amino group and an EMCS reagent (N-(6-Maleimidocaproyloxy) succinimide: manufactured by Dojin Co., Ltd.). The SH group can be introduced into the DNA by using 5'-Thiol-Modifier C6 (manufactured by Glen Research Co., Ltd.).

Furthermore, combinations of functional groups for use in fixation include, for example, a combination of an epoxy group (on solid phase) and an amino group (end of nucleic acid probe) in addition to the combination of the thiol group and the maleimide group described above. Furthermore, surface treatments with various kinds of silane coupling agents are also effective, and oligonucleic acid having introduced a functional group capable of reacting with a functional group introduced by the silane coupling agent may also be used. Further, a method of coating a resin having functional groups is also effective.

The surface of the DNA micro-array 404 after undergoing the hybridization reaction is washed to remove nucleic acid not bound to the probe, and is then (usually) dried. Thereafter, the substrate of the DNA micro-array is irradiated with excitation light to measure the fluorescence intensity (406). Furthermore, the substrate is scanned while being irradiated with excitation light to obtain a scan image (407) proportional to the fluorescence intensity.

[1-2. Outline of hybridization reaction]

The outline of the hybridization reaction (405) described above will now be described using FIG. 3. FIG. 3 shows the state of the hybridization reaction on the DNA micro-array. In the organism, the DNA has a double-helical structure in most cases, bonding between the double strands is achieved by hydrogen bonding between bases. On the other hand, the RNA often exists in a single state. Types of bases include four types of ACGT for the DNA and four types of ACGU for the RNA, and a pair of bases capable of hydrogen bonding is an A-T(U) pair and a G-C pair, respectively.

The hybridization reaction generally refers to a state in which single-stranded nucleic acid molecules are partially bound together via partial base sequences therein. Furthermore, in this embodiment, a nucleic acid molecule (probe 301) bound to the substrate on the upper side in FIG. 3 is shorter than a nucleic acid molecule (302) in a sample on the lower side. Thus, if the nucleic acid molecule existing in the sample contains a base sequence of the probe, this hybridization reaction proceeds successfully, and the target nucleic acid molecule in the sample is trapped in the DNA micro-array.

[1-3. Principle of DNA micro-array]

The principle of the DNA micro-array that is used for determining bacteria of the infectious disease will now be described using FIG. 5. The DNA micro-array (500-1, 500-2) shown in FIG. 5 is one example of the DNA micro-array fabricated for the purpose of determining Staphylococcus aureus.

The left sequence in this figure is a treatment sequence derived from a Staphylococcus aureus wild strain when the DNA micro-array is used, and the right sequence is a treatment sequence derived from an Escherichia coli wild strain. For example, the left sequence may be considered as a flow of treatment of blood of a patient infected with Staphylococcus aureus, while the right sequence may be considered as a flow of treatment of blood of a patient infected with Escherichia coli.

In any case, the same treatment is carried out in principle. That is, DNA is first extracted from, for example, blood or expectoration of the patient infected with bacteria (501-1, 501-2). At this time, generally, DNA derived from body cells of the patient may be included.

Furthermore, if the amount of extracted DNA is small, it is amplified by the PCR method or the like. At this time, a fluorescent substance or substance capable of having a fluorescent substance bound thereto is generally incorporated as a label (502-1, 502-2).

If DNA is not amplified, a fluorescent substance or substance capable of having a fluorescent substance bound thereto is incorporated as a label while forming a complementary strand using the extracted DNA (503-1, 503-2). Alternatively, a fluorescent substance or substance capable of having a fluorescent substance bound thereto is added directly to the extracted DNA as a label.

Usually, if PCR amplification is performed, and it is intended for determination of bacteria of the infectious disease, a site of the base sequence constituting ribosome RNA called 16s rRNA is commonly amplified. In this case, the PCR primer for Staphylococcus aureus on the left is almost same as the PCR primer for Escherichia coli on the right. More specifically, multiplex PCR is carried out using a primer set capable of amplifying a site coding 16s rRNA of any type of bacteria. In this case, consequently, both right and left hybridization solutions in FIG. 5 contain a plurality of types of base sequences. The reason for this will be described in detail with a subsequent figure.

On the other hand, if it is desired to carry out more detailed sequence analysis, for example, the PCR primer set for Staphylococcus aureus and the PCR primer set for Escherichia coli are set separately. In this case, if the primer is set so that only a specified site of a genome of bacteria is selectively amplified, the types of base sequences contained in the hybridization solution are considerably limited. Nevertheless, because several types of strains of bacteria generally exist in the natural world, it is unlikely that only one type of base sequence exists in the hybridization solution.

If the DNA micro-array designed for the purpose of determining Staphylococcus aureus correctly operates, a spot positively reacts (500-1) for the left hybridization solution, and a spot negatively reacts (500-2) for the right hybridization solution.

Just in the same manner, if the DNA micro-array designed for the purpose of determining Escherichia coli correctly operates, a spot negatively reacts for the left hybridization solution, and a spot positively reacts for the right hybridization solution. In this embodiment, infectious bacteria are determined using a DNA micro-array in which several types of spots reacting uniquely with various bacteria are arranged together.

[1-4. Reason why a plurality of base sequences exist]

The reason why a plurality of types of base sequences exist in the hybridization solution of FIG. 5 will now be described using FIG. 6. Normally, bacteria existing in the natural world frequently mutate. As a result, several types of main strains surviving through selection may exist at the same time. For example, appearance of strains causing a problem in hospital infection and the like is ascribable to the fact that bacteria, which normally have no chemical resistance, acquire chemical resistance through mutation. As a result of acquiring chemical resistance, bacteria having a strong reproductive power appear even in an environment where disinfection efforts are conducted. In this way, the base sequence of same bacteria has variations of several types.

In FIG. 6, the genome structures of two types of strains: Mu50 and MW2 in Staphylococcus aureus are shown. The strains are different in the total number of base groups in the genome, with one having 2,878,040 base groups and the other having 2,820,462 base groups. Further, the site coding 16s ribosome RNA exists at total five locations: two locations in the forward direction and three locations in the backward direction for Mu50, while the site exists at total six locations: three locations in the forward direction and three locations in the backward direction for MW2.

The base sequences of the respective sites of 16s ribosome RNA are very similar, but not identical, to one another. That is, even if only one type of strain of bacteria existing in the body of a patient infected with bacteria is to be examined, a plurality of types of similar base sequences exist in the hybridization solution if the hybridization solution is adjusted by a general treatment shown in FIG. 5. The main object of the biological species determination method in this application is to allow determination of biological species with stability even if an experiment using a DNA micro-array is conducted for the plurality of similar nucleic acids.

[1-5. Examples of hybridization reaction experiment]

Specific Examples of a hybridization reaction experiment conducted for the purpose of determining causative bacteria of an infectious disease will now be described in detail. Furthermore, the biological species determination method according to the present invention is not limited to determination of causative bacteria of the infectious disease described below, but may be used for determination on physical constitutions of human beings such as MHC, and analysis of DNA and RNA related to diseases such as cancers.

[1-5-1. Preparation of probe DNA]

Nucleotide sequences (I-n) (n is an integer number) shown in Table 1 were designed as a probe for detection of Enterobacter cloacae.

Specifically, probe base sequences shown below were selected from genome sites coding 16s rRNA. The probe base sequence group is designed so that it is highly specific for the bacteria, and can be expected to have a hybridization sensitivity sufficient and free from variations "where possible" with each probe base sequence.

Table.1

| | |
|---|---|
| I-1 | CAgAgAgCTTgCTCTCgggTgA |
| I-2 | gggAggAAggTgTTgTggTTAATAAC |
| I-3 | ggTgTTgTggTTAATAACCACAgCAA |
| I-4 | gCggTCTgTCAAgTCggATgTg |
| I-5 | ATTCgAAACTggCAggCTAgAgTCT |
| I-6 | TAACCACAgCAATTgACgTTACCCg |
| I-7 | gCAATTgACgTTACCCgCAgAAgA |

The probe shown in the table was synthesized as a functional group to be fixed on a DNA micro-array, and then a thiol group was introduced into the 5' terminal of nucleic acid according to an established method. After the functional group was introduced, the probe was purified, and freeze-dried. The freeze-dried probe for internal control was stored in a freezer at -30°C.

Probe sets shown below (Tables 2-1 to 2-9) were designed in the same manner for Staphylococcus aureus (A-n), Staphylococcus epidermidis (B-n), Escherichia coli (C-n), Pneumobacillus (D-n), Pseudomonas aeruginosa (E-n), Serratia (F-n), Streptococcus pneumoniae (G-n), Influenza bacillus (H-n) and Enterococcus faecalis (J-n) (n is an integer number).

Table.2-1

| | |
|---|---|
| A-1 | gAACCgCATggTTCAAAAgTgAAAgA |
| A-2 | CACTTATAgATggATCCgCgCTgC |
| A-3 | TgCACATCTTgACggTACCTAATCAg |
| A-4 | CCCCTTAgTgCTgCAgCTAACg |
| A-5 | AATACAAAgggCAgCgAAACCgC |
| A-6 | CCggTggAgTAACCTTTTAggAgCT |
| A-7 | TAACCTTTTAggAgCTAgCCgTCgA |
| A-8 | TTTAggAgCTAgCCgTCgAAggT |
| A-9 | TAgCCgTCgAAggTgggACAAAT |

Table.2-2

| | |
|---|---|
| B-1 | gAACAgACgAggAgCTTgCTCC |
| B-2 | TAgTgAAAgACggTTTTgCTgTCACT |
| B-3 | TAAgTAACTATgCACgTCTTgACggT |
| B-4 | gACCCCTCTAgAgATAgAgTTTTCCC |
| B-5 | AgTAACCATTTggAgCTAgCCgTC |
| B-6 | gAgCTTgCTCCTCTgACgTTAgC |
| B-7 | AgCCggTggAgTAACCATTTgg |

Table.2-3

| | |
|---|---|
| C-1 | CTCTTgCCATCggATgTgCCCA |
| C-2 | ATACCTTTgCTCATTgACgTTACCCg |
| C-3 | TTTgCTCATTgACgTTACCCgCAg |
| C-4 | ACTggCAAgCTTgAgTCTCgTAgA |
| C-5 | ATACAAAgAgAAgCgACCTCgCg |
| C-6 | CggACCTCATAAAgTgCgTCgTAgT |
| C-7 | gCggggAggAAgggAgTAAAgTTAAT |

Table.2-4

| | |
|---|---|
| D-1 | TAgCACAgAgAgCTTgCTCTCgg |
| D-2 | TCATgCCATCAgATgTgCCCAgA |
| D-3 | CggggAggAAggCgATAAggTTAAT |
| D-4 | TTCgATTgACgTTACCCgCAgAAgA |
| D-5 | ggTCTgTCAAgTCggATgTgAAATCC |
| D-6 | gCAggCTAgAgTCTTgTAgAgggg |

Table.2-5

| | |
|---|---|
| E-1 | TgAgggAgAAAgTggggg ATCTTC |
| E-2 | TCAgATgAgCCTAggTCggATTAgC |
| E-3 | gAgCTAgAgTACggTAgAgggTgg |
| E-4 | gTACggTAgAgggTggTggAATTTC |
| E-5 | gACCACCTggACTgATACTgACAC |
| E-6 | TggCCTTgACATgCTgAgAACTTTC |
| E-7 | TTAgTTACCAgCACCTCgggTgg |
| E-8 | TAgTCTAACCgCAAgggggACg |

Table.2-6

| | |
|---|---|
| F-1 | TAgCACAgggAgCTTgCTCCCT |
| F-2 | AggTggTgAgCTTAATACgCTCATC |
| F-3 | TCATCAATTgACgTTACTCgCAgAAg |
| F-4 | ACTgCATTTgAAACTggCAAgCTAgA |
| F-5 | TTATCCTTTgTTgCAgCTTCggCC |
| F-6 | ACTTTCAgCgAggAggAAggTgg |

Table.2-7

| | |
|---|---|
| G-1 | AgTAgAACgCTgAAggAggAgCTTg |
| G-2 | CTTgCATCACTACCAgATggACCTg |
| G-3 | TgAgAgTggAAAgTTCACACTgTgAC |
| G-4 | gCTgTggCTTAACCATAgTAggCTTT |
| G-5 | AAgCggCTCTCTggCTTgTAACT |
| G-6 | TAgACCCTTTCCggggTTTAgTgC |
| G-7 | gACggCAAgCTAATCTCTTAAAgCCA |

Table.2-8

| | |
|---|---|
| H-1 | gCTTgggAATCTggCTTATggAgg |
| H-2 | TgCCATAggATgAgCCCAAgTgg |
| H-3 | CTTgggAATgTACTgACgCTCATgTg |
| H-4 | ggATTgggCTTAgAgCTTggTgC |
| H-5 | TACAgAgggAAgCgAAgCTgCg |
| H-6 | ggCgTTTACCACggTATgATTCATgA |
| H-7 | AATgCCTACCAAgCCTgCgATCT |
| H-8 | TATCggAAgATgAAAgTgCgggACT |

Table.2-9

| | |
|---|---|
| J-1 | TTCTTTCCTCCCgAgTgCTTgCA |
| J-2 | AACACgTgggTAACCTACCCATCAg |
| J-3 | ATggCATAAgAgTgAAAggCgCTT |
| J-4 | gACCCgCggTgCATTAgCTAgT |
| J-5 | ggACgTTAgTAACTgAACgTCCCCT |
| J-6 | CTCAACCggggAgggTCATTgg |
| J-7 | TTggAgggTTTCCgCCCTTCAg |

[1-5-2. Preparation of PCR Primer for amplification of specimens]

Nucleotide sequences shown in Table 2 were designed as PCR Primer for amplification of 16s rRNA nucleic acid (target nucleic acid) for detecting causative bacteria.

Specifically, a probe set for amplifying uniquely a genome site coding 16s rRNA, that is a primer with unique melting temperatures equated where possible at both ends of the 16s rRNA coding area of about 1500 base length was designed. Furthermore, a plurality of types of primers were designed so that variants and a plurality of 16s rRNA coding areas existing on the genome could be amplified.

| | Primer No. | Sequence |
|---|---|---|
| Forward Primer | F-1 | 5' GCGGCGTGCCTAATACATGCAAG 3' |
| | F-2 | 5' GCGGCAGGCCTAACACATGCAAG 3' |
| | F-3 | 5' GCGGCAGGCTTAACACATGCAAG 3' |
| Reverse Primer | R-1 | 5' ATCCAGCCGCACCTTCCGATAC 3' |
| | R-2 | 5' ATCCAACCGCAGGTTCCCCTAC 3' |
| | R-3 | 5' ATCCAGCCGCAGGTTCCCCTAC 3' |

Primers shown in the table were synthesized and then purified by high performance liquid chromatography (HPLC), three types of Forward Primers and three types of Reverse Primers were mixed, and the mixture was dissolved in a TE buffer solution so that the concentration of each primer was 10 pmol/μl as a final concentration.

[1-5-3. Extraction of Enterobacter_cloacae Genome DNA (model specimen)]

[1-5-3-1 Culture of microorganisms & pretreatment of Genome DNA]

First, an Enterobacter cloacae standard strain was cultured according to an established method. 1.0 ml (OD600=0.7) of the microorganism culture solution was put in a 1.5 ml micro-tube, and bacteria were collected by centrifugal separation (8500 rpm, 5 min, 4°C).

The supernatant was removed, 300 μl of enzyme buffer (50 mM Tris-HCL: pH 8.0, 25 mM EDTA) was then added to the solution, and the solution was re-suspended using a mixer. The re-suspended bacteria solution was subjected to centrifugal separation to collect bacteria again (8500 rpm, 5 min, 4°C).

The supernatant was removed, the enzyme solutions described below were added to the collected bacteria, and the solution was re-suspended using a mixer.

| | |
|---|---|
| Lysozyme | 50 μl (20 mg/ml in Enzyme Buffer) |
| N-Acetylmuramidase SG | 50 μl (0.2 mg/ml in Enzyme Buffer) |

Then, the bacteria solution prepared by adding the enzyme solutions to the bacteria and re-suspending the same was left standing in an incubator at 37°C for 30 minutes to dissolve cell walls.

[1-5-3-2 Genome DNA extraction]

Genome DNA extraction of microorganisms shown below was carried out using a nucleic acid purification kit (MagExtractor -Genome-: manufactured by TOYOBO Co., Ltd.).

Specifically, first, 750 μl of dissolving and adsorbing solution and 40 μl of magnetic beads were added to a pretreated microorganism suspension, and the resultant solution was vigorously stirred for 10 minutes using a tube mixer (step 1).

Then, the micro-tube was set on a stand for separation (magical trapper), and left standing for 30 seconds to collect magnetic particles on the wall surface of the tube, and the supernatant was removed with the tube set on the stand (step 2).

Then, 900 μl of cleaning liquid was added to the solution, and the resultant solution was stirred with a mixer for about 5 seconds to be re-suspended (step 3).

Then, the micro-tube was set on the stand for separation (magical trapper), and left standing for 30 seconds to collect magnetic particles on the wall surface of the tube, and the supernatant was removed with the tube set on the stand (step 4).

After Steps 3 and 4 were repeated and second cleaning was performed (step 5), 900 μl of 70% ethanol was added to the solution, and the resultant solution was stirred with a mixer for about 5 seconds to be re-suspended (step 6).

Then, the micro-tube was set on the stand for separation (magical trapper), and left standing for 30 seconds to collect magnetic particles on the wall surface of the tube, and the supernatant was removed with the tube set on the stand (step 7).

After steps 6 and 7 were repeated and second cleaning with 70% ethanol was performed (step 8), 100 μl of pure water was added to the collected magnetic particles, and the resultant solution was stirred with the tube mixer for 10 minutes.

Then, the micro-tube was set on the stand for separation (magical trapper), and left standing for 30 seconds to collect magnetic particles on the wall surface of the tube, and the supernatant was collected in a new tube with the tube set on the stand.

[1-5-3-3 Inspection of collected Genome DNA]

The Genome DNA of the collected microorganism (Enterobacter cloacae strain) was subjected to agarose electrophoresis and absorbance measurement at 260/280 nm according to an established method, and its quality (content of low-molecular nucleic acid and degree of degradation) and collected amount were examined.

In this Example, about 10 μg of Genome DNA was collected, and degradation of Genome DNA and existence of rRNA were not found. The collected Genome DNA was dissolved in a TE solution so that its final concentration would be 50 ng/μl, and was used in the following Example.

[1-5-4. Fabrication of DNA micro-array]

[1-5-4-1 Cleaning of glass substrate]

A glass substrate of synthetic quartz (size: 25 mm × 75 mm × 1 mm, manufactured by Iiyama Tokushu Glass Co., Ltd.) was placed in a heat-resistant and alkali-resistant rack, and immersed in a cleaning liquid for ultrasonic cleaning, adjusted to have a predetermined concentration. The glass substrate was immersed in the cleaning liquid all the night through, and then ultrasonically cleaned for 20 minutes. Subsequently, the substrate was taken out from the liquid, lightly rinsed with pure water, and then ultrasonically cleaned in ultra-pure water for 20 minutes. Then, the substrate was immersed in a 1 N aqueous sodium hydroxide solution heated to 80°C for 10 minutes. The substrate was washed with pure water and ultra-pure water again to prepare a quartz glass substrate for DNA chips.

[1-5-4-2 Surface treatment]

A silane coupling agent KBM-603 (manufactured by Shin-Etsu Silicones) was dissolved in pure water so that its concentration would be 1%, and stirred at room temperature for 2 hours. Subsequently, the previously washed glass substrate was immersed in the silane coupling agent solution, and left standing at room temperature for 20 minutes. The glass substrate was taken out from the solution, the surface thereof was lightly rinsed with pure water, and nitrogen gas was then blown to the both surfaces of the substrate to dry the substrate. Then, the dried substrate was baked for 1 hour in an oven heated to 120°C, thus completing the coupling agent treatment to introduce an amino group into the substrate surface. Then, an EMCS solution, obtained by dissolving N-(6-maleimidocaproyloxy)succinimido (hereinafter abbreviated as EMCS) (manufactured by Dojindo Corporate) in a mixed solvent of dimethyl sulfoxide and methanol (1:1) so that its final concentration would be 0.3 mg/ml, was prepared. The glass substrate after being baked was naturally cooled, and immersed in the prepared EMCS solution at room temperature for 2 hours. By this treatment, the amino group introduced into the surface of the glass substrate reacted with a succinimide group of EMCS to introduce a maleimide group into the surface of the glass substrate. The glass substrate taken out from the EMCS solution was washed using the mixed solvent having EMCS dissolved therein, further washed with ethanol, and then dried under an atmosphere of nitrogen gas.

[1-5-4-3 probe DNA]

The microorganism detecting probe fabricated in this Example was dissolved in pure water, the resultant solution was dispensed so that each would have a final concentration (when dissolved in ink) of 10 μM, and then freeze-dried, and water was removed from the solution.

[1-5-4-4 Discharge of DNA by BJ printer and binding to substrate]

An aqueous solution containing 7.5 wt% of glycerin, 7.5 wt% of thioglycol, 7.5 wt% of urea and 1.0 wt% of Acetylenol EH (manufactured by Kawaken Fine Chemicals Co., Ltd.) was prepared. Subsequently, the previously prepared seven types of probes (Table 1) were dissolved in the above mixed solvent so as to obtain specified concentrations. The obtained DNA solution was filled in an ink tank for Bubble Jet® Printer (trade name: BJF-850 manufactured by Canon Inc.), and the ink tank was mounted on a print head.

Furthermore, the Bubble Jet Printer® used here is a printer modified so as to allow printing on a flat plate. Furthermore, in this Bubble Jet Printer® , about 5 picoliters of DNA solution can be spotted at about 120 micrometer pitches by imputing a print pattern according to a specified file creation process.

Subsequently, using this modified Bubble Jet Printer® , a printing operation was performed on one glass substrate to fabricate an array. After ensured that printing was reliably performed, the array was left standing in a humidifying chamber for 30 minutes to allow the maleimide group on the surface of the glass substrate to react with the thiol group at the end of the nucleic acid probe.

[1-5-4-5 Cleaning]

After the reaction proceeded for 30 minutes, the DNA solution remaining on the surface was washed away with 10 mM phosphate buffer solution (pH 7.0) containing 100 mM of NaCl to obtain a DNA micro-array with single-stranded DNA fixed on the surface of the glass substrate.

[1-5-5. Amplification and labeling of specimen (PCR amplification & capturing of fluorescence label)]

Amplification of microorganism DNA as a specimen and the labeling reaction are as follows.

| | | |
|---|---|---|
| Premix PCR Reagent (TAKARA ExTaq) | 25μl | |
| Template Genome DNA | 2μl | (100ng) |
| Forward Primer mix | 2μl | (20pmol/tube each) |
| Reverse Primer mix | 2μl | (20pmol/tube each) |
| Cy-3 dUTP (1mM) | 2μl | (2nmol/tube) |
| H20 | 17μl | |
| Total | 50μl | |

The reaction solution described above was made to undergo an amplification reaction by a commercially available thermal cycler according to the following protocol.

| | | |
|---|---|---|
| 95℃ | 10 min. | |
| 92℃ | 45 sec. | 35 Cycles |
| 55℃ | 45 sec. | |
| 72℃ | 45 sec. | |
| 72℃ | 10 min. | |

After the reaction was completed, the primer was removed using a column for purification (QIAGEN QIAquick PCR Purification Kit), and then the amplified product was quantitatively determined, and determined to be a labeled specimen.

[1-5-6. Hybridization]

The DNA micro-array fabricated in the "1-5-4. Fabrication of DNA micro-array" and the labeled specimen fabricated in the "1-5-5. Amplification and labeling of specimen (PCR amplification & capturing of fluorescence label)" were used to carry out a detection reaction.

[1-5-6-1 Blocking of DNA micro-array]

BSA (bovine serum albumin Fraction V: manufactured by Sigma Co., Ltd.) was dissolved in a 100 mM NaCl/ 10 mM phosphate buffer, and the DNA micro-array fabricated in "1-5-4. fabrication of DNA micro-array" was immersed in this solution at room temperature for 2 hours to perform blocking. After blocking was completed, the DNA micro-array was washed with a 2xSSC solution (Nacl 300 mM, Sodium Citrate (trisodium citrate dihydrate, $C_6H_5Na_3 \cdot 2H_2O$) 30 mM, pH 7.0) containing 0.1 wt% SDS (sodium dodecyl sulfate), then rinsed with pure water, and then drained by a spin-drying apparatus.

[1-5-6-2 Hybridization]

The drained DNA micro-array was set in a hybridization apparatus (Genomic Solution Inc. Hybridization station), and made to undergo a hybridization reaction using a hybridization solution and hybridization conditions shown below ([1-5-6-3], [1-5-6-4]).

[1-5-6-3 Hybridization solution]

6x SSPE/10% Form amide/Target ($2^{nd}$ PCR Products total amount) (6xSSPE: NaCl 900 mM, $NaH_2PO_4 \cdot H_2O$ 60 mM, EDTA 6 mM, pH 7.4)

[1-5-6-4 Hybridization conditions]

65°C 3 min → 92°C 2 min → 45°C 3 hr → Wash 2xSSC/0.1% SDS at 25°C → Wash 2xSSC at 20°C → (Rinse with $H_2O$ Manual) → Spin dry (The DNA micro-array was made to undergo a hybridization reaction at 65°C for 3 minutes, 92°C for 2 minutes, and 45°C for 3 hours, then washed with 2xSSC/0.1% SDS at 25°C, washed with 2xSSC at 20°C, rinsed with pure water, and spin-dried.)

[1-5-7. Detection of microorganisms (fluorometry)]

The DNA micro-array after completion of the hybridization reaction was subjected to fluorometry using a fluorescence detecting apparatus for DNA micro-arrays (GenePix 4000B manufactured by Axon Co., Ltd.).

One example of a scan image obtained as a result of the Examples described above is shown in FIG. 7. Furthermore, in FIG. 7, a probe having higher fluorescence intensity is shown with a darker color.

Reference numeral 701 denotes a scan image obtained by making the DNA micro-array react with a sample containing a genome of Staphylococcus aureus, and reference numeral 702 denotes one example of a scan image obtained by making the DNA micro-array react with a sample containing a genome of Escherichia coli.

The alphabets described on the left in the figure are alphabets for the probe sequence, and probes A to J are bound uniquely to Staphylococcus aureus (A), Staphylococcus epidermidis (B), Escherichia coli (C), Pneumobacillus (D), Pseudomonas aeruginosa (E), Serratia (F), Streptococcus pneumoniae (G), Influenza bacillus (H), Enterobacter cloacae (I) and Enterococcus faecalis (J), respectively.

2. Description of biological species determination processing (step S103)

Biological species determination processing (step S103) carried out using a scan image obtained at the step S101 will now be described.

[2-1. System configuration]

FIG. 2 is a block diagram showing the configuration of an information processing apparatus for realizing an information processing method (biological species determination method) according to one embodiment of the present invention.

The biological species determination method is realized in an apparatus comprised of an external storage device 201, a central processing unit (CPU) 202, a memory 203 and an input/output device 204. The external storage device 201 retains programs for realizing the biological species determination method according to this embodiment, and scan images obtained as a result of hybridization reactions. Furthermore, it has a function of retaining biological species determination results derived in this embodiment. The central processing unit (CPU) 202 executes programs of the biological species determination method, and controls all devices. The memory 203 temporarily stores programs, subroutines and data processed in the central processing unit (CPU) 202. The input/output device 204 interacts with a user. Furthermore, an execution trigger of the program is inputted by the user via the input/output device 204. Further, the user can view the determination result and set a parameter of the program via the input/output device 204.

[2-2. Outline of general biological species determination processing]

Before describing the information processing method of the present invention in detail, a specific example of the conventional determination processing method for a scan image obtained by the hybridization reaction is shown, and its problems are reviewed in order to define the feature of the present method.

[2-2-1. Determination processing by homology search]

As previously described with U.S. Patent No. 6040138 in "BACKGROUND OF THE INVENTION", there is the method of making a determination on existence of a plurality of types of causative bacteria by homology search for determining a biological species of an unknown sample.

The methods include, for example, an analysis method carried out by an expression analysis using a DNA micro-array, that is an analysis method in which in FIG. 7, the average fluorescence intensity of a plurality of probes of each group, from probes of A group to probes of J group, is determined to be the fluorescence intensity of targeted bacteria, and then the existence probability of each type of bacteria is defined as {(fluorescence intensity of X group) /(total sum of the fluorescence intensities of A to J groups)} (X is one of A to J). According to the analysis method, even if a plurality of types of causative bacteria exist in an unknown sample, the existence probability of each type of bacteria can be appropriately determined.

However, as previously described in "BACKGROUND OF THE INVENTION", if the analysis method is used, existence/nonexistence of causative bacteria having similar base sequences cannot be determined accurately. For showing one example, ideally, the result of the hybridization reaction is such that only probes of line A of the scan image 701 have increased fluorescence intensities, and only probes of line C of the scan image 702 have increased fluorescence intensities (this ideal result of the scan image 701 is same as the experimental result shown in FIG. 5).

Here, if all probes have an ideal nature as shown in FIG. 5, the average fluorescence intensity of probes of each group, from probes of A group to probes of J group, can be determined to be the intensity of targeted bacteria and in this case, since the experimental result for one sample has ten values of fluorescence intensities of A to J groups, the existence probability of each type of bacteria can be defined as {(fluorescence intensity of X group)/(total sum of fluorescence intensities of A to J groups)} (X is one of A to J).

As shown in FIG. 7, however, such an ideal result is not actually obtained, but so called a "cross hybridization reaction" occurs, and probes other than those of line A have increased fluorescence intensities in the case of the scan image 701, and probes other than those of line C have increased fluorescence intensities in the case of the scan image 702. Further, in the case of the scan image 702, probes having low fluorescence intensities may exist in the line C.

In this way, the method by determining the existence probability is suitable in quantitative analysis of mRNA using a conventional DNA micro-array, but is not suitable in determination of causative bacteria of an infections disease leading to the result shown in FIG. 7.

[2-2-2. Other methods]

For determining a biological species using the result of the hybridization reaction, several methods can be considered in addition to the above homology search. They include, for example, a method in which scan images obtained as a result of the hybridization reaction for reference samples consisting of known biological species are stored in advance, and pattern recognition is performed based on the scan images from the reference samples of the known biological species to determine a biological species of an unknown sample.

Now, realizability of determination of biological species by pattern recognition will be discussed below. Here, in particularly, a method in which for each of the reference sample and the unknown sample, the fluorescence intensities of all probes are expressed collectively as one vector, and vectors for both samples are used to determine the biological species is discussed.

For example, if fluorescence intensities shown in the example in FIG. 7 are obtained, total 72 proves of A to J groups are expressed collectively as one 72-dimensional vector. That is, one vector (referred to as "integrated vector") is obtained from the experimental result for one sample (reference sample or unknown sample). A plurality of integrated vectors obtained from a plurality of reference samples are compared with an integrated vector obtained from the unknown sample, whereby the biological species to which the unknown sample corresponds can be determined by pattern recognition.

An example of pattern recognition in a 2-dimensional vector (that is integrated vector derived based on a scan image obtained using a DNA micro-array having 2 probes (X, Y)) is shown in FIGS. 8A and 8B (at this time, both the probe X and probe Y are probes designed in the expectation that they would be unique for Staphylococcus aureus).

In FIGS. 8A and 8B, total 64 samples are given as reference samples, and 64 integrated vectors are obtained from the result of measurement thereof (in these figures, the values of X and Y axes indicate the fluorescence intensities of probes X and Y, respectively (in fact, they are values obtained by normalizing the measured values of the samples)). Of 64 samples, for example, the integrated vector derived from Staphylococcus aureus is expressed as a black spot, and the integrated vector derived from Escherichia coli is expressed as a white spot. Furthermore, two types of probes are used here for convenience of explanation, but many types usually exist as shown in FIG. 7, and the measurement result is a high-dimensional vector.

Pattern recognition can be performed using a classification tree (FIG. 8A) for a distribution chart of integrated vectors derived from reference samples shown in FIG. 8B.

The classification tree refers to a method of dividing a feature space having reference samples distributed hierarchically, and if the classification tree is created with a set of reference samples shown in FIG. 8B, for example, a structure shown in FIG. 8A is obtained. Each Node of the classification tree shown in FIG. 8A indicates a border shown with a thick line in FIG. 8B, and the feature space can be divided into 7 partial spaces as a whole. The partial spaces correspond to leaf nodes of the classification tree, and they are shown by white and black circles. Consequently, in the case of the example shown in FIGS. 8A and 8B, there are 4 partial spaces for Staphylococcus aureus, and 3 partial spaces for Escherichia coli.

In the determination method using the classification tree, when an integrated vector derived from an unknown sample is given, which partial space the integrated vector belongs to is determined, and a biological species corresponding to the partial space to which it belongs is determined (In the case of FIGS. 8A and 8B, if the value of X is 0.5 or smaller and the value of Y is 0.5 or smaller, for example, the integrated vector belongs to an area in which integrated vectors derived from Staphylococcus aureus are distributed. Furthermore, if the value of X is 0.5 or smaller, and the value of Y is 0.75 or greater, the integrated vector belongs to an area in which integrated vectors derived from Escherichia coli are distributed).

By tracing the classification tree hierarchically, which partial space the integrated vector derived from the unknown sample belongs to can be determined, and therefore a biological species can be generally determined very fast.

As apparent from the above description, when pattern recognition is performed using integrated vectors, at least one reference samples should be prepared per one biological species, and if the number of reference samples is small, accuracy of determination of the biological species is reduced or determination is impossible. Of course, no problem arises if only one type of causative bacteria exists, but a plurality of types of causative bacteria usually exist in the case of determination of causative bacteria of an infectious disease, and in this case, correct determination results can no longer be obtained. This is because the size and the direction of the integrated vector vary depending on the combination of causative bacteria contained in a specimen undergoing the hybridization reaction. That is, if a plurality of types of causative bacteria exist in the specimen, the derived integrated vector approximates to a sum of results for a plurality of types of causative bacteria, and pattern recognition based on this integrated vector leads to the determination result such that the unknown sample is similar to none of reference samples.

For coping with this problem, it is necessary that many reference samples combined with various types of causative bacteria be prepared for one biological species, and the result of carrying out the hybridization reaction for each reference sample be stored. However, it is not practical to prepare reference samples for all combinations of causative bacteria. Thus, it is not appropriate to apply such pattern recognition directly to biological species determination where causative bacteria having similar base sequences are included.

[2-3. Feature of biological species determination processing based on this application]

Under the background described above, in biological species determination processing based on this application, the determination method by pattern recognition is employed for solving the problems in "2-2-1. Homology search", while the above discussion about problems associated with use of pattern recognition is considered, so that causative bacteria can be accurately determined for similar base sequences even if all combinations of causative bacteria are not prepared. Specifically, the biological species determination processing is characterized in that vector filtering processing is added in vector comparison between the reference sample and the unknown sample. Detailed description is presented below.

[2-4. Flow of biological species determination processing based on this application]

FIG. 9 is a functional block diagram for explaining processes of the biological species determination method according to this embodiment. Reference numeral 901 denotes a "scan image for the reference sample", which is obtained as a result of making the reference sample containing a nucleic acid fragment derived from a targeted biological species undergo the hybridization reaction. A labeling molecule such as a fluorescence substance is usually added to this nucleic acid fragment, so that the intensity of the hybridization reaction with the DNA micro-array can easily be measured.

Reference numeral 902 denotes a hybridization reaction digitizing section, which carries out processing for digitizing the intensity of the hybridization reaction between the DNA micro-array and the reference sample. Reference numeral 903 denotes a measurement result vectoring section, which carries out processing for statistically processing measurement values for probes on the DNA micro-array obtained in the hybridization reaction digitizing section and then re-expressing the values as an n-dimensional vector. Reference numeral 904 denotes a vector normalizing section, which normalizes a produced vector. Vectored and normalized data (reference vector data) is stored in a reference vector data collecting section 905.

Reference numeral 909 denotes a main component analyzing section, which performs analysis of main components for reference vector data stored in the reference vector data collecting section 905. Results of analysis of main components in the main component analyzing section 909 are used in filter processing in a vector filtering section 905.

Reference numeral 907 denotes a "scan image for an unknown sample", which is digitized in the hybridization reaction digitizing section 902 as in the case of the scan image for the reference sample, and converted into a vector expression in the measurement result vectoring section 903, and normalized in the vector normalizing section 904 (unknown vector data).

A biological species determining section 908 determines a biological species of an unknown sample by patter recognition, and uses a vector filtered in the vector filtering section 905 for determination of the biological species. That is, vector data filtered in the vector filtering section 905 for normalized unknown vector data obtained from the scan image for the unknown sample is compared with vector data filtered in the vector filtering section 905 for reference vector data stored in the reference vector data collecting section 906 to determine the biological species of the unknown sample.

[2-5. Detailed description of processing]

Processing in each section in FIG. 9 will be described in detail below.

[2-5-1. Vector normalization processing]

Vector normalization processing in the vector normalizing section 904 refers to processing for normalizing a vector derived based on the fluorescence intensity obtained for each sample.

For example, if a plurality of spots of the same probe exist on a DNA micro-array, the average value of the fluorescence intensities of the spots is generally determined to be the fluorescence intensity of the probe.

An example of a DNA micro-array having a plurality of spots of the same type is shown in FIG. 10. In the DNA micro-array of FIG. 10, 4 sets of 20 types of probes are fixed on a substrate, and thus total 80 spots exist. In this case, the average intensity of 4 probes of the same type is determined to be a measurement value for the probe, and collectively expressed as a 20-dimensional vector.

In the example of the DNA micro-array shown in FIG. 10, the probe at the upper left is a positive control. For example, nucleic acid interfering with no probe is previously spotted as a positive control, and a substance with a fluorescent pigment added to oligonucleotide serving as a complementary strand of a probe base sequence thereof is added just before the hybridization reaction. Furthermore, a probe having a partial base sequence always contained in the sample may be a positive control.

In the experiment of the DNA micro-array, the fluorescence intensity may increase or decrease as a whole. In this case, the fluorescence intensities of all probes can be normalized by using the positive control described above. Furthermore, there is a method in which the fluorescence intensities of all probes are normalized using as a reference the measurement value of the spot having the highest fluorescent luminance in the DNA micro-array.

[2-5-2. Vector filtering processing]

Vector filtering processing in the vector filtering section 905 characterizing the present invention will now be described. First, the concept of vector filtering processing will be described. As described above, the integrated vector obtained as a result of the hybridization reaction is determined for each sample in a multidimensional space determined by the number of probes. At this time, the integrated vector in the multidimensional space significantly varies depending on the type of causative bacteria contained in the specimen.

For example, the integrated vector obtained from the result of the reaction of a specimen containing only one type of causative bacterial is very different from the integrated vector obtained from the result of the reaction of a specimen containing other types of causative bacteria in addition to the above causative bacteria.

That is, a combination of causative bacteria contained in a specimen, for which the result of the reaction is used as a reference sample, significantly influences the result of determination. Thus, a method in which reference samples obtained as a result of hybridization reactions including all combinations of causative bacteria are prepared can be considered, but this method is not practical as described previously.

Thus, the present invention is characterized by determining causative bacteria with the influence of the combination being avoided where possible. Specifically, the integrated vector is filtered to extract a vector component (specified vector) of predetermined causative bacteria from the unknown sample, while a specified vector of predetermined causative bacteria is similarly extracted for the integrated vector of the reference sample. The specified vectors are compared with each other to perform pattern recognition, whereby existence/nonexistence of the predetermined causative bacteria can be accurately determined while reducing the number of reference samples.

In this way, vector filtering processing is carried out to extract a specified vector excluding vector components other than the vector component of predetermined causative bacteria, whereby a correct determination result can be obtained only by preparing limited reference samples.

Specific processing in the vector filtering section 905 will now be described. The most primitive algorithm of the vector filtering section 906 is shown in FIG. 11. Reference numeral 1101 denotes a filter for Staphylococcus aureus, in which the coefficient of the black spot is 1 and the coefficient of the white spot is 0. For example, if the result of the hybridization reaction shown in FIG. 7 is obtained, a filter 1101 is applied to both experimental data 701 and experimental data 702. Consequently, the 72-dimensional vector is filtered into a 9-dimensional vector.

Similarly, reference numeral 1102 denotes a filter for Escherichia coli, in which the coefficient of the black spot is 1 and the coefficient of the white spot is 0. If this filter is applied to, for example, experimental data shown in FIG. 7, the 72-dimensional vector is filtered into a 7-dimensional vector. Consequently, if experimental results for two reference samples as shown in FIG 7 are obtained, two 9-dimensional vectors and two 7-dimensional vectors are collected in the reference vector data collecting section 906 of FIG. 9. On the other hand, from experimental data for the unknown sample, one 9-dimensional specified vector and one 7-dimensional specified vector are obtained, and they are compared with the reference vectors of the same dimensional to estimate existence/nonexistence of Staphylococcus aureus and Escherichia coli, respectively. In this way, a filter is prepared for each biological species, such as a filter for Staphylococcus aureus and a filter for Escherichia coli shown in FIG. 10, and the vector is filtered with the filter, and a specified vector is determined, whereby existence/nonexistence for each biological species is determined by pattern recognition.

[2-5-3. Main component analysis processing]

In FIG. 10, an example of simple filtering in which measurement values corresponding to respective biological species probes are multiplied by 1, and other probes are multiplied by 0 is shown for convenience of explanation but generally, a filter can be achieved by multiplying the measurement value of each probe by a constant between 0 and 1 based on a finding obtained previously. Now, a method for building the filter will be described below.

Generally, techniques for compressing information of vector groups include main component analysis (for details, for example see "Regression Analysis and Main Component Analysis, Statistical Analysis Program Course 2 written by Toshiro Houga and Shigeji Hashimoto" published by JUSE Press, Ltd.; ISBN:4817120118; (1980/05)). In this method, a convariance matrix obtained from a plurality of measurement result vectors is subjected to characteristic value resolution, and a characteristic vector corresponding to each characteristic value is used to subject the measurement result vector to main component resolution (spectral resolution). Since the characteristic vector can be converted into a normalized orthogonal basis, main component resolution (spectral resolution) is so-called orthogonal coordinate conversion. Accordingly, the dimension of the vector before main component resolution is basically same as the dimension of the vector after main component resolution. However, after main component resolution, components corresponding to extremely small characteristic values can be often ignored and in this case, the dimension of the vector can be reduced. This functions as a filter.

Processing in the main component analysis section 909 for performing main component analysis for each biological species and building a filter will be described below.

FIG. 12 is a flowchart showing the flow of processing in the main component analysis section 909 of FIG. 9. First, biological species constituting a filter are selected. In the case of determination of causative bacteria of an infectious disease, for example, Staphylococcus aureus, Escherichia coli and the like are designated. Then, of integrated vectors of reference samples collected in the reference vector data collecting section 906 of FIG. 9, only those derived from biological species samples selected at step S1201 are selected. Usually, at this time, integrated vector data of reference samples derived from biological species selected at step S1201 is all selected. Furthermore, integrated vector data of reference samples derived from biological species other than the biological species selected at step S1201 is selected.

Then, a convariance matrix of a group of integrated vector data selected at steps S1202 and S1203 is determined to calculate characteristic values, and components for small characteristic values are ignored. In this way, measurement values for probes important for determining the biological species selected at step S1201 are filtered.

For example, if there are n probes, the convariance matrix obtained from a group of integrated vector data selected at steps S 1202 and S1203 is a symmetrical non-negative matrix of nxn, and n characteristic values exist therein. If characteristic values arranged in descending order are represented by λi (i=1, 2, ···, n), the accumulated proportion of mth and lower-order components is calculated by the following formula.

$$\sum_{i=1}^{m} \lambda_i \Big/ \sum_{i=1}^{n} \lambda_i$$

At the time when this reaches, for example, 80%, main component resolution (spectral resolution) is stopped, and components corresponding to characteristic values smaller than this value are ignored. Furthermore, the value, that is 80% in the example described above, may be set to any percentage given by a user. Furthermore, individual characteristic vectors may be displayed to the user together with characteristic values, allowing the user to select main component resolution components to be ignored.

At this time, all integrated vector data of reference samples derived from biological species other than biological species selected at step S1201 may be selected, but if a large number of biological species should be determined, a situation arises in which the number of integrated vectors selected at step S1203 is much larger than the number of integrated vectors selected at step S1202. Consequently, the result of subsequent main component analysis is affected by the integrated vectors selected at step S1203, so that the result of main component analysis may be almost the same for all biological species. For avoiding this problem, for example, some contrivance is made so that the number of integrated vectors selected at step S1203 is almost equal to the number of integrated vectors selected at step S1202.

For example, integrated vector data is selected from integrated vector data of reference samples derived from biological species other than biological species selected at step S1201 in a number equal to the number of integrated vectors selected at step S1202. At this time, if the number of biological species to be determined is N, for example, integrated vectors of reference samples in a number equal to 1/(N-1) of the number of integrated vectors collected for each biological species are randomly selected in order to select integrated vector data of reference samples for various biological species. Consequently, the number of integrated vectors selected at step S1203 becomes almost equal to the number of integrated vectors selected at step S1202.

[2-5-4. Determination processing]

Generally, comparison and classification among vectors are performed using a technique called "pattern recognition". Details of the technique are reviewed in, for example, the literature of "Statistical Pattern Recognition: A Review" Anil K. Jain, Robert P.W. Duin, and Jianchan Mao in IEEE Transaction on Pattern Analysis and Machine Learning, Vol. 22, No. 1, January 2000, pp. 4-pp. 37. Any of the k-Nearest-Neighbor method, the classification tree, the Support Vector Machine, the Bayes determination method, the boosting method, the neural net method and the like may be applied to the biological species determination method of the present invention.

Now, pattern recognition by the k-Nearest-Neighbor method and pattern recognition using the classification tree will be described. As pattern recognition using the classification tree has been described, the k-Nearest-neighbor method is described here. The k-Nearest-Neighbor method is the most primitive method of algorisms of pattern recognition. The k-Nearest-Neighbor method is a method in which a distance between the specified vectors of the reference sample and the unknown sample is calculated, and the biological species of the reference sample to which the unknown sample is close in distance is determined to be a determination result in principle. For the distance between the specified vectors, the Euclidean distance expressed by the following formula is generally used.

$$\sqrt{\sum_{i=1}^{n} (x_i - y_i)^2 \Big/ n} \quad \left(\begin{array}{l}\text{X is the vector derived from the unknown sample.}\\ \text{Y is the vector derived from the reference sample.}\end{array}\right)$$

Furthermore, the absolute distance expressed by the following formula may be used.

$$\sum_{i=1}^{n} |x_i - y_i| / n$$

The k-Nearest-Neighbor method is a method in which reference samples are arranged in order of increasing distance, and the biological species for which the number of reference samples is the largest of K reference samples from the nearest side is determined to be a determination result. In particular, the 1-Nearest-Neighbor method is a method in which the biological species of the reference sample closest to the specified vector derived from the unknown sample is determined to be a determination result.

Pattern recognition using the classification tree will now be described using FIGS. 13 to 15. First, the outline of pattern recognition using the classification tree will be described using FIG. 13. As shown in FIG. 13, in pattern recognition using the classification tree, classification tree creation processing for creating a plurality of classification trees 1303 from a learning pattern 1301 is first carried out (1302).

The learning pattern 1301 is referred to as so-called "supervised data", and a category to which the pattern belongs is known. In the case of determination of causative bacteria of an infections disease, for example, the pattern includes a hybridization pattern of a DNA chip and information of bacteria thereof in a pair. Furthermore, a step of creating the classification tree 1303 is generally referred to as a learning phase.

A pattern belonging to an unknown category (unknown pattern 1304) is pattern-matched using the classification tree 1303 created in the learning phase (1305) to estimate a category it belongs to. In the case of determination of causative bacteria of an infections disease, for example, the bacteria are determined based on the hybridization pattern of the DNA chip. A step of pattern-matching the unknown pattern 1304 is generally referred to as a pattern recognition phase.

In this embodiment, a plurality of classification trees 1303 are created from the same learning pattern 1301 in the learning phase. If n classification trees 1303 are created in the learning phase, then n recognition results are obtained in correspondence with respective classification trees 1303 in the pattern recognition phase. A final recognition result 1306 is determined by decision of majority in these n recognition results. Furthermore, if the each created classification result 1303 is a classification result including a probability, all n recognition results with probability are summed for each category to define the category of highest probability as the overall recognition result 1306 (this algorithm is generally referred to as an ensemble algorithm).

Details of the above classification tree creation processing (1302) will now be described using FIG. 14. FIG. 14 is a flowchart showing a flow of classification tree creation processing (1302). As shown in this figure, for creating the classification tree, a route node is first set to a current node as initial setting at step S1401. Here, the current node is a node that is currently perceived, and includes a subset of the learning pattern (may include a full set or null set). Furthermore, the route node is a first parent node of the classification tree, and includes all learning patterns.

Then at step S1402, whether the current node includes the learning pattern is determined. If the current node is the route node, it always includes the learning pattern, but if the classification tree creation processing proceeds to break down the classification tree, so that a node of a lower layer becomes the current node, no learning pattern may be included in the current node. If no learning pattern is included in the current node, processing proceeds to step S1408, where the current node is determined to be a NULL node. Here, the NULL node is a node in which the determination result is unknown, and if the unknown pattern falls into the NULL node, the category existence probability of its parent node is determined to be a pattern recognition result.

If the current node is set to the NULL node, no more child nodes are required to be created for the node, and therefore processing returns to the parent node of the node (that is the parent node of the node is set to the current node (step S1410)).

On the other hand, if the current node includes the learning pattern (the current node is not the NULL node), whether the current node meets the requirement of a leaf node is checked at step S1403. The leaf node refers to a node having no child node, and for checking is, for example, an entropy of the learning pattern included in the current node is determined, and if the entropy is equal to or less than a threshold, the current node is determined to be the leaf node. Furthermore, if the threshold that is used at this time has been set to 0, then the leaf node includes only a learning pattern belonging to a single category.

If it is determined that the current node meets the requirement of the leaf node as a result of checking at step S1403, processing proceeds to step S1409, where the current node is determined to be the leaf node. Because the leaf node has no child node as described above, no child node is generated for the node, and therefore processing returns to the parent node of the node (that is the parent node of the node is set to the current node (step S1410)).

On the other hand, if the current node is neither the NULL node nor the leaf node, a determination function of the node is determined at step S1404, and child nodes are generated (step S1405). At step S1407, the child nodes are set to current nodes one after another, and processing at steps S1402 to S1405 and steps S1408 to S1410 is repeated to create the classification tree further deeply.

Furthermore, in this embodiment, a two-branched classification tree is used for the classification tree, and two child nodes are generated at step S1405 on every occasion (this algorithm is referred to as a two-branched classification ensemble algorithm). If the algorithm is implemented with a program, setting of the current node to the child node is realized by a loop, and two-branches involve two loops.

If all nodes under the route node are developed, that is the ends of all nodes under the route node become NULL nodes or leaf nodes (if "No" at step S1406), processing proceeds to step S1411, where whether the parent node is the route node or not is checked. If the current node is located at a node of a lower layer of the classification tree at the time when all child nodes under the route node are developed, Steps S1411, S1410 and S1406 are repeated, whereby the current node moves to nodes in upper layers, and at the time when the current node moves to a node immediately below the route node, the processing is completed.

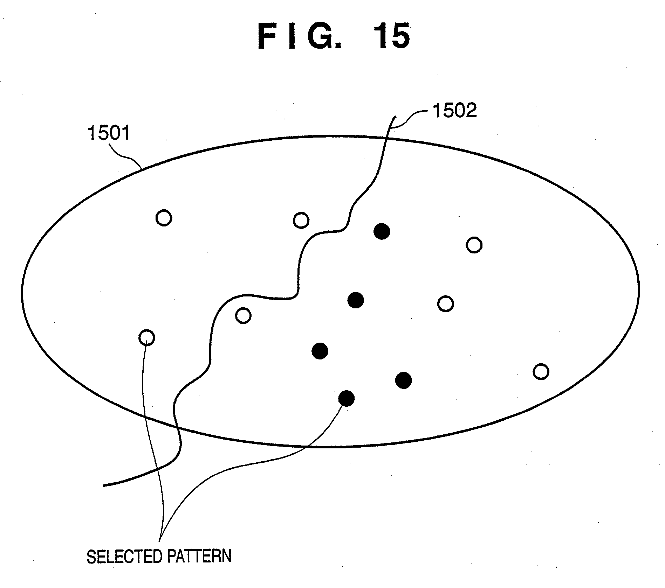

The outline of a step of determining the determination function in the node (step S1404) will now be described using FIG. 15. The classification tree creation processing (1302) described with FIG. 14 is common to the classification tree creation algorithm. In order that the classification tree created in individual classification tree creation processing obtains a high recognition rate, the type of algorithm with which the determination function is determined in each node is important. In this embodiment, the determination function is determined using random sampling.

FIG. 15 is a view for explaining the outline of processing for the determination function using random sampling, and reference numeral 1501 denotes a distribution of learning patterns included in the current node. Here, for the sake of simplicity, there are 2 types of categories expressed by white and black, respectively, and the current node includes total 12 learning patterns, that is 7 white learning patterns and 5 black learning patterns.

For determining the determination function, first, one learning pattern is randomly selected from all learning patterns included in the current node, and then one learning pattern is randomly selected from learning patterns of another category different from the category to which the above selected learning pattern belongs (reference numeral 1503 denotes the selected learning pattern). A function a function indicating which of these two learning patterns is more similar is determined to be determination function.

For the "degree of similarity" serving as an indicator for determining the function indicating which of two learning patterns is more similar, the Euclidean distance is commonly used, but it is not limited to the Euclidean distance, and any distance scale capable of constituting a distance space may be used. If the learning pattern employs the Euclidean distance with vectors, a determination curve 1502 defined by the determination function has a hyperplane. Generally, if more complicated distance scale is used, a more complicated determination curve is obtained. Furthermore, in the case of FIG. 15, the right side of the determination curve 1502 is an area close to the black learning pattern, and the left side of the determination curve 1502 is an area close to the white learning pattern.

Furthermore, the number of learning patterns that are randomly selected is not limited to 2, but the determination function may be determined by selecting m learning patterns from one category and selecting m learning patterns from the category, determining an average pattern of m average patterns for each category, and measuring the distances from these 2 patterns.

In this way, in classification tree creation processing in this embodiment, a two-branched classification tree ensemble algorithm is used, and the determination function in each node of the classification tree is defined by a function indicating which of two leaning patterns mutually different in category, randomly selected from learning data existing in each node, is more similar, whereby a high recognition rate can be realized in the created classification tree.

3. Conclusion

As apparent from the above description, in this embodiment, a biological species is determined using a hybridization reaction with a DNA-micro-array instead of the conventional culture method, whereby the biological species is determined easily, inexpensively and in a short time.

Furthermore, for determining the biological species using a scan image obtained as a result of the hybridization reaction, the biological species is determined using pattern recognition based on comparison with reference samples instead of the conventional method of determining an existence probability, whereby even biological species having similar base sequences can be determined.

At this time, in comparison with the reference sample, a specified vector obtained by extracting predetermined causative bacteria components from an integrated vector is used, rather than using an integrated vector obtained by integrating fluorescence intensities of all probes on the reference sample, whereby the biological species can be determined even if the number of reference samples prepared in advance is small.

Furthermore, a vector filter for each type of causative bacteria that is used for extracting the specified vector from the integrated vector is acquired by subjecting integrated vectors of a plurality of reference samples containing predetermined causative bacteria to main component analysis.

Furthermore, the present invention may be applied to a system comprised of a plurality of devices (for example host computer, interface device, reader, printer, etc.), or applied to an apparatus comprised of one device (for example copier, facsimile apparatus, etc.).

Furthermore, the object of the present invention is achieved by supplying to a system or apparatus a storage medium in which program codes of software achieving functions of the embodiment described previously, and causing a computer (or CPU or MPU) of the system or apparatus to read and execute the program code stored in the storage medium, as a matter of course.

In this case, the program code itself read from the storage medium realizes the function of the embodiment described previously, and the storage medium storing the program code constitutes the present invention.

Storage media for supplying program codes may include, for example, floppy® disks, hard disks, optical disks, magneto-optical disks, CD-ROMs, CD-Rs, magnetic tapes, nonvolatile memory cards and ROMs.

Furthermore, by executing the program code read by the computer, not only the function of the embodiment described previously is achieved, but also the OS (operating system) or the like operating on the computer performs part of all of actual processing based on instructions of the program code and by the processing, the function of the embodiment described previously is realized, as a matter of course.

Further, the program code read from the storage medium is written in a memory provided in an expanded capability board inserted in the computer or an expanded capability unit connected to the computer, then the CPU or the like provided in the expanded capability board or expanded capability unit performs part or all of actual processing based on the instructions of the program code and by the processing, the function of the embodiment described previously is achieved, as a matter of course.

The present invention is not limited to the above embodiments and various changes and modifications can be made within the spirit and scope of the present invention. Therefore to apprise the public of the scope of the present invention, the following claims are made.

## Claims

**1.** An information processing apparatus for processing information about the signal intensity of each probe on a DNA micro-array obtained by subjecting a predetermined specimen to a hybridization reaction using the DNA micro-array where said each probe is arranged, said each probe representing a nucleic acid complimentary to a partial nucleotide sequence of a nucleic acid of biological species, the information processing apparatus **characterized by** comprising:

retaining means for retaining first information as information about the signal intensity of said each probe obtained by subjecting any known biological species to the hybridization reaction;
acquiring means for acquiring second information as information about said signal intensity of said each probe obtained by subjecting said predetermined specimen to the hybridization reaction;
extracting means for extracting information related to a predetermined biological species from said first and second information; and
determining means for determining whether or not said predetermined specimen may comprise the predetermined biological species based on information related to the predetermined biological species, extracted from said first information by said extracting means, and information related to the predetermined biological species from said second information.

2. The information processing apparatus according to claim 1, **characterized in that** said first and second information is vector data having the signal intensities of the probes as components in a multidimensional space corresponding to the number of said probes on said DNA micro-array.

3. The information processing apparatus according to claim 2, **characterized in that** said extracting means performs conversion to reduce the number of dimensions of the vector data composed of said first and second information.

4. The information processing apparatus according to claim 2, **characterized in that** said extracting means extracts information related to said predetermined biological species based on main component analysis carried out for a plurality of vector data which includes said predetermined biological species and forms a part of the vector data of first information retained by said retaining means.

5. The information processing apparatus according to claim 1, **characterized in that** said determining means makes a determination by pattern recognition processing.

6. The information processing apparatus according to claim 5, **characterized in that** said determination means is a classification tree, the classification tree is created using a two-branched classification ensemble algorithm, and a determination function in each node of the classification tree is defined by a function indicating which of two learning patterns different in category, randomly selected from learning data existing in the each node, is more similar.

7. An information processing method for processing information about the signal intensity of each probe on a DNA micro-array obtained by subjecting a predetermined specimen to a hybridization reaction using the DNA micro-array where said each probe is arranged, said each probe representing a nucleic acid complementary to a partial nucleotide sequence of biological species, the information processing method **characterized by** comprising:

a retaining step of retaining first information as information about the signal intensity of said each probe obtained by subjecting any known biological species to the hybridization reaction;
an acquiring step of acquiring second information as information about said signal intensity of said each probe obtained by subjecting said predetermined specimen to the hybridization reaction;
an extracting step of extracting information related to a predetermined biological species from said first and second information; and
a determining step of determining whether or not said predetermined specimen may comprise the predetermined biological species based on information related to the predetermined biological species, extracted from said first information by said extracting means, and information related to the predetermined biological species from said second information.

8. The information processing method according to claim 7, **characterized in that** said first and second information is vector data having the signal intensities of the probes as components in a multidimensional space corresponding to the number of said probes on said DNA micro-array.

9. The information processing method according to claim 8, **characterized in that** in said extracting means, conversion is performed to reduce the number of dimensions of the vector data being said first and second information.

10. The information processing method according to claim 8, **characterized in that** in said extracting step, information related to said predetermined biological species is extracted based on main component analysis carried out for a plurality of vector data which includes said predetermined biological species and forms a part of the vector data being first information retained in said retaining step.

11. The information processing method according to claim 7, **characterized in that** in said determining step, a determination is made by pattern recognition processing.

12. The information processing method according to claim 11, **characterized in that** said determination step is carried out with the classification tree, the classification tree is created using a two-branched classification ensemble algorithm, and a determination function in each node of the classification tree is defined by a function indicating which of two learning patterns different in category, randomly selected from learning data existing in the each node, is more similar.

**13.** A control program for realizing the information processing method according to any one of claims 7 to 12 by a computer.

**14.** A recording medium storing a control program for realizing the information processing method according to any one of claims 7 to 12 by a computer.

FIG. 1

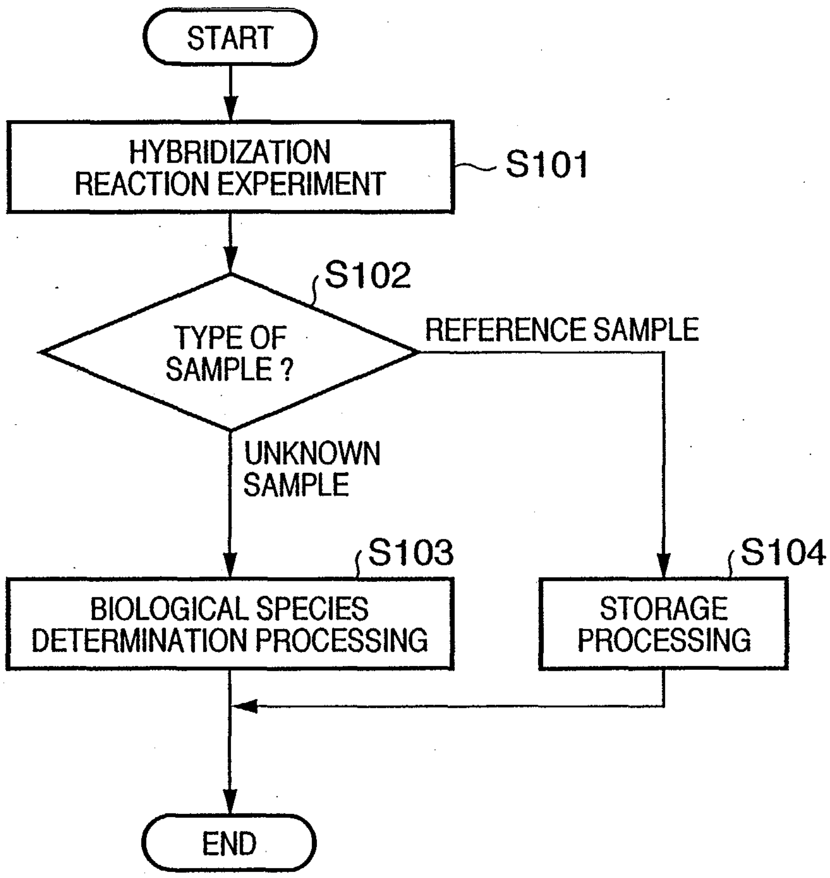
START
HYBRIDIZATION REACTION EXPERIMENT
S101
S102
TYPE OF SAMPLE ?
REFERENCE SAMPLE
UNKNOWN SAMPLE
S103
S104
BIOLOGICAL SPECIES DETERMINATION PROCESSING
STORAGE PROCESSING
END

FIG. 2

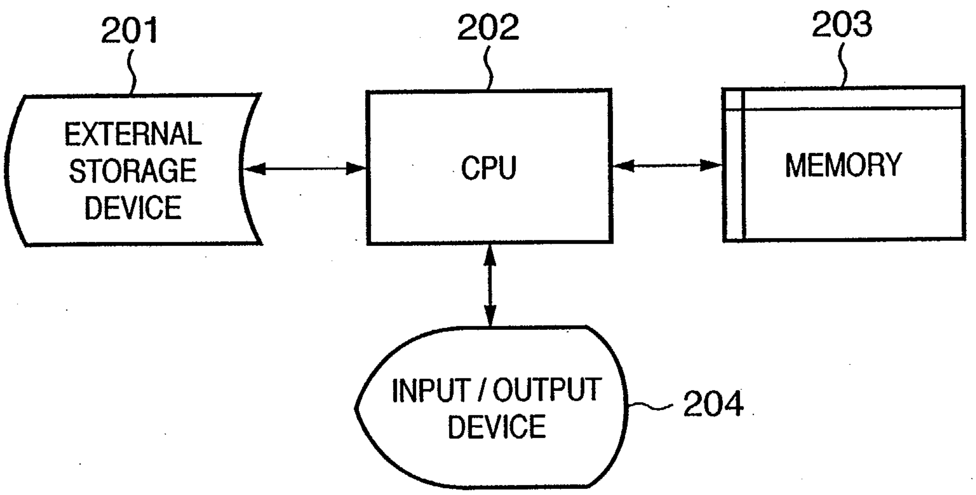
201
EXTERNAL STORAGE DEVICE
202
CPU
203
MEMORY
INPUT / OUTPUT DEVICE
204

FIG. 3

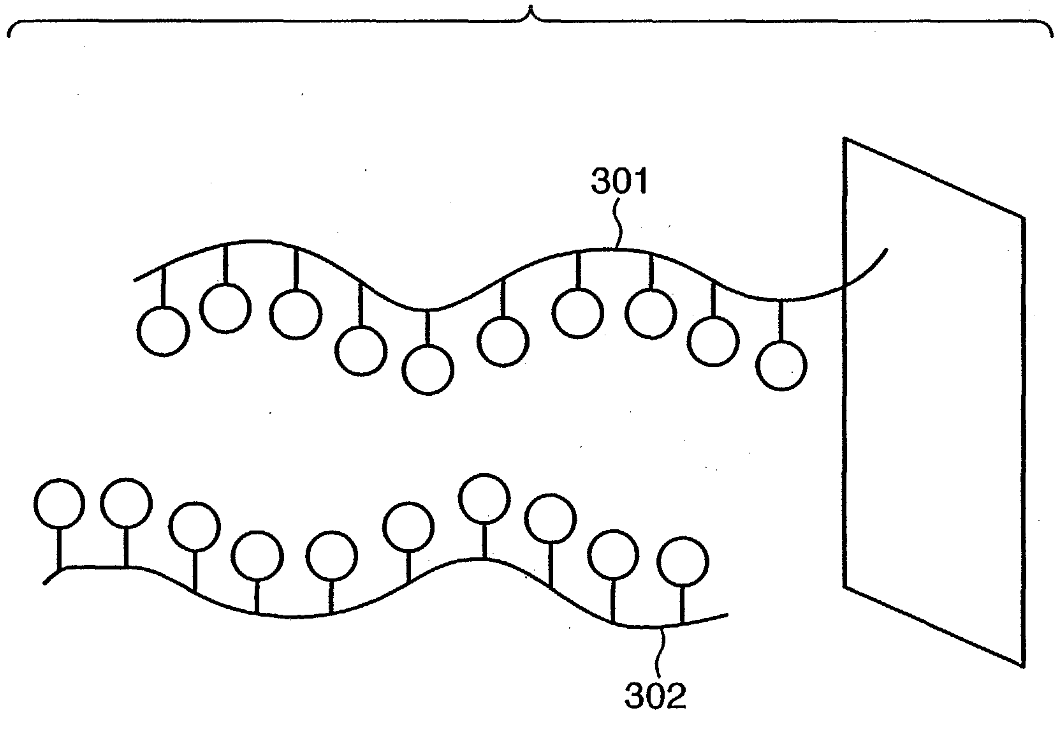

FIG. 4

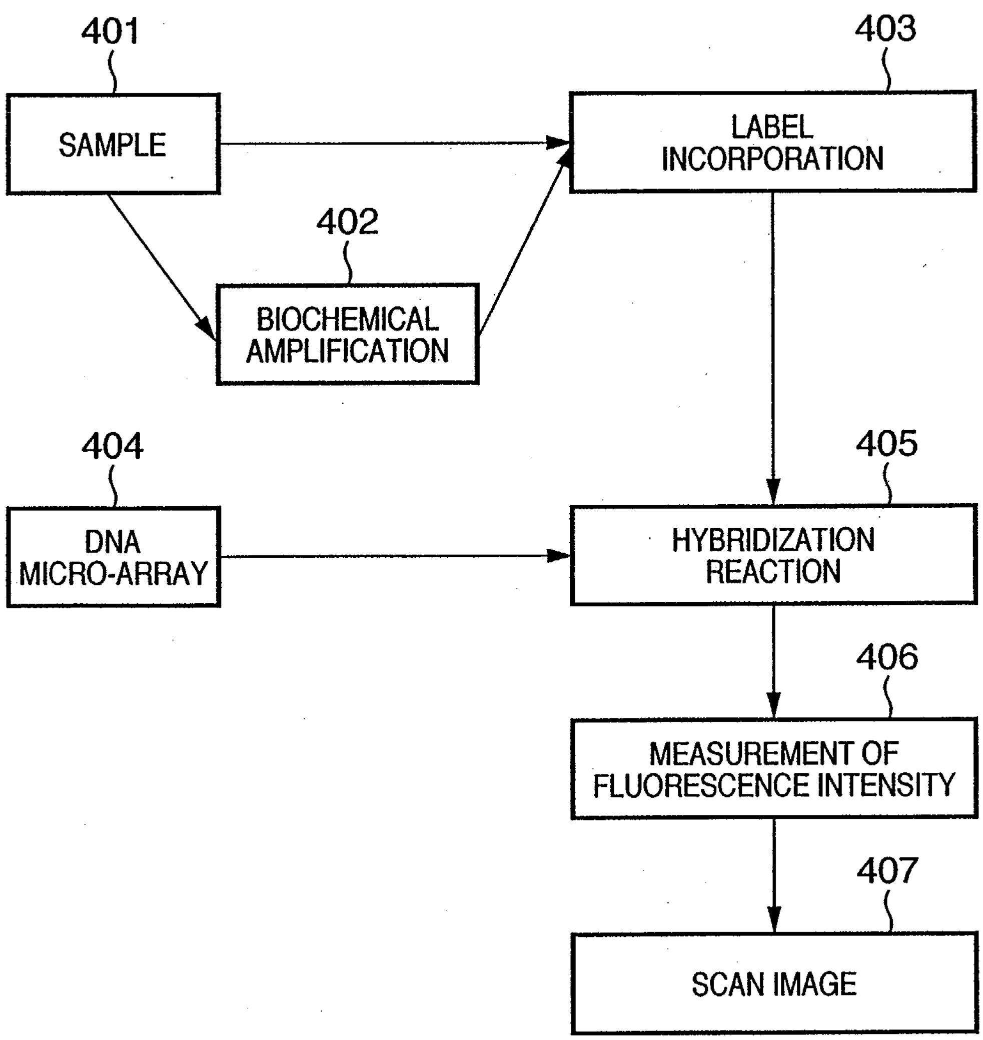
401
SAMPLE
402
BIOCHEMICAL AMPLIFICATION
403
LABEL INCORPORATION
404
DNA MICRO-ARRAY
405
HYBRIDIZATION REACTION
406
MEASUREMENT OF FLUORESCENCE INTENSITY
407
SCAN IMAGE

FIG. 5

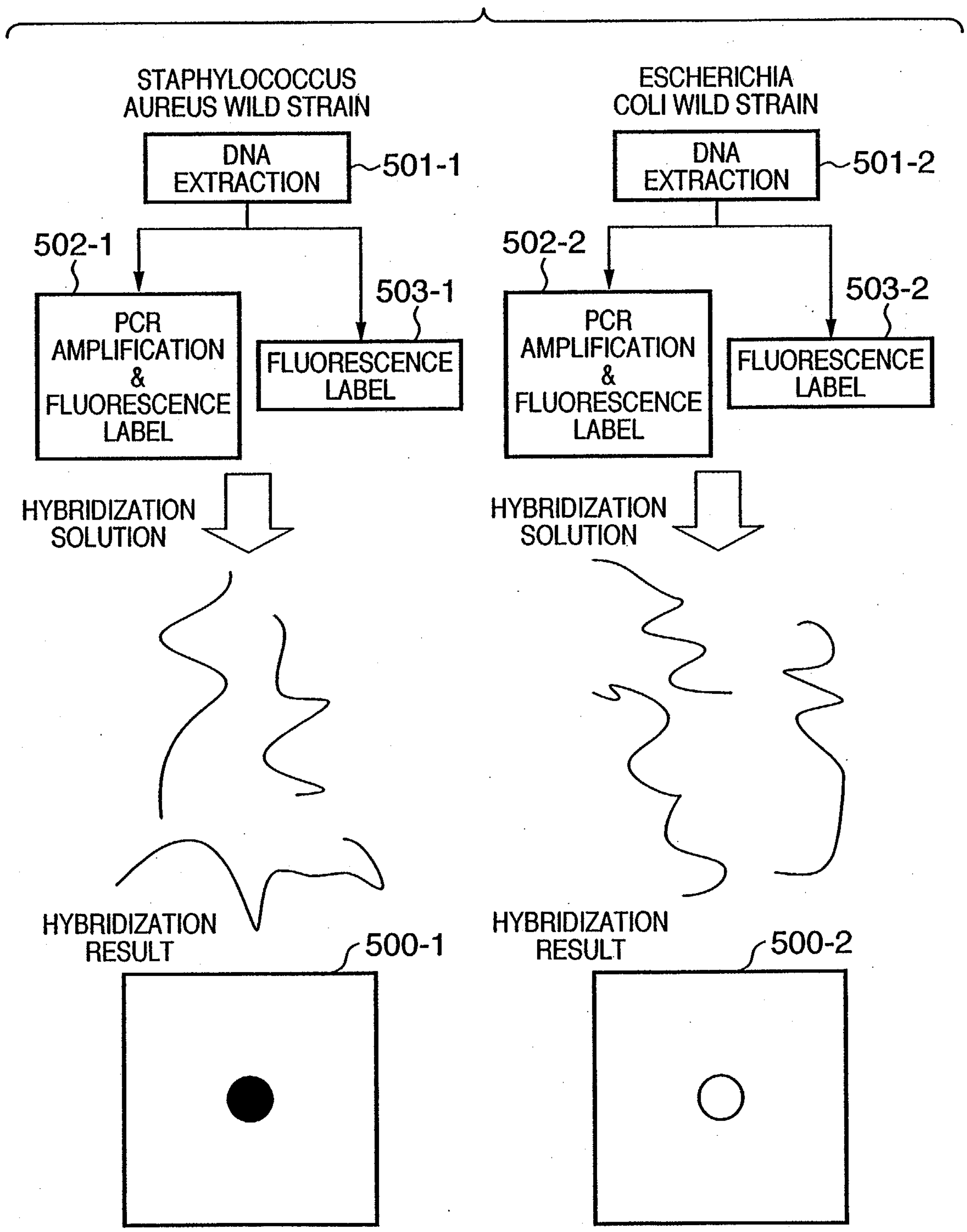
STAPHYLOCOCCUS AUREUS WILD STRAIN
DNA EXTRACTION
501-1
502-1
PCR AMPLIFICATION & FLUORESCENCE LABEL
503-1
FLUORESCENCE LABEL
HYBRIDIZATION SOLUTION
HYBRIDIZATION RESULT
500-1
ESCHERICHIA COLI WILD STRAIN
DNA EXTRACTION
501-2
502-2
PCR AMPLIFICATION & FLUORESCENCE LABEL
503-2
FLUORESCENCE LABEL
HYBRIDIZATION SOLUTION
HYBRIDIZATION RESULT
500-2

FIG. 6

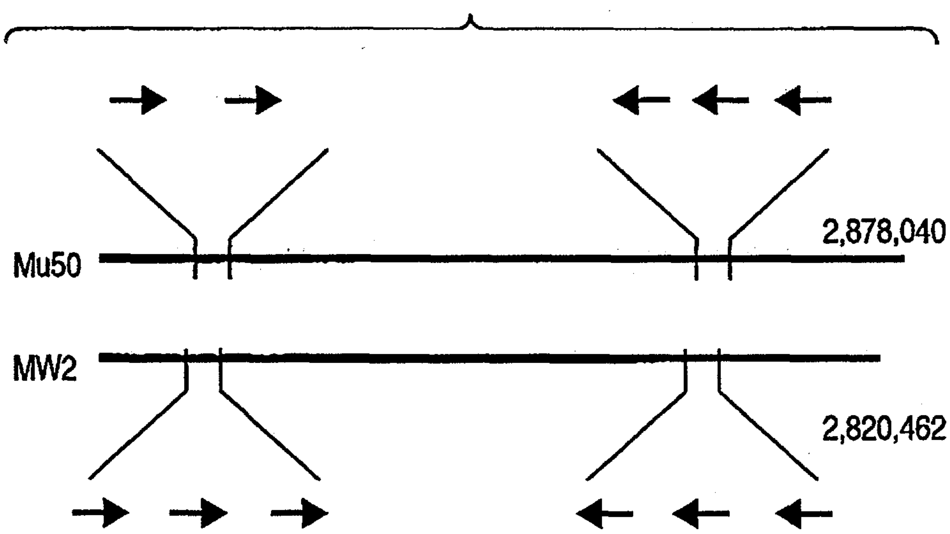

FIG. 7

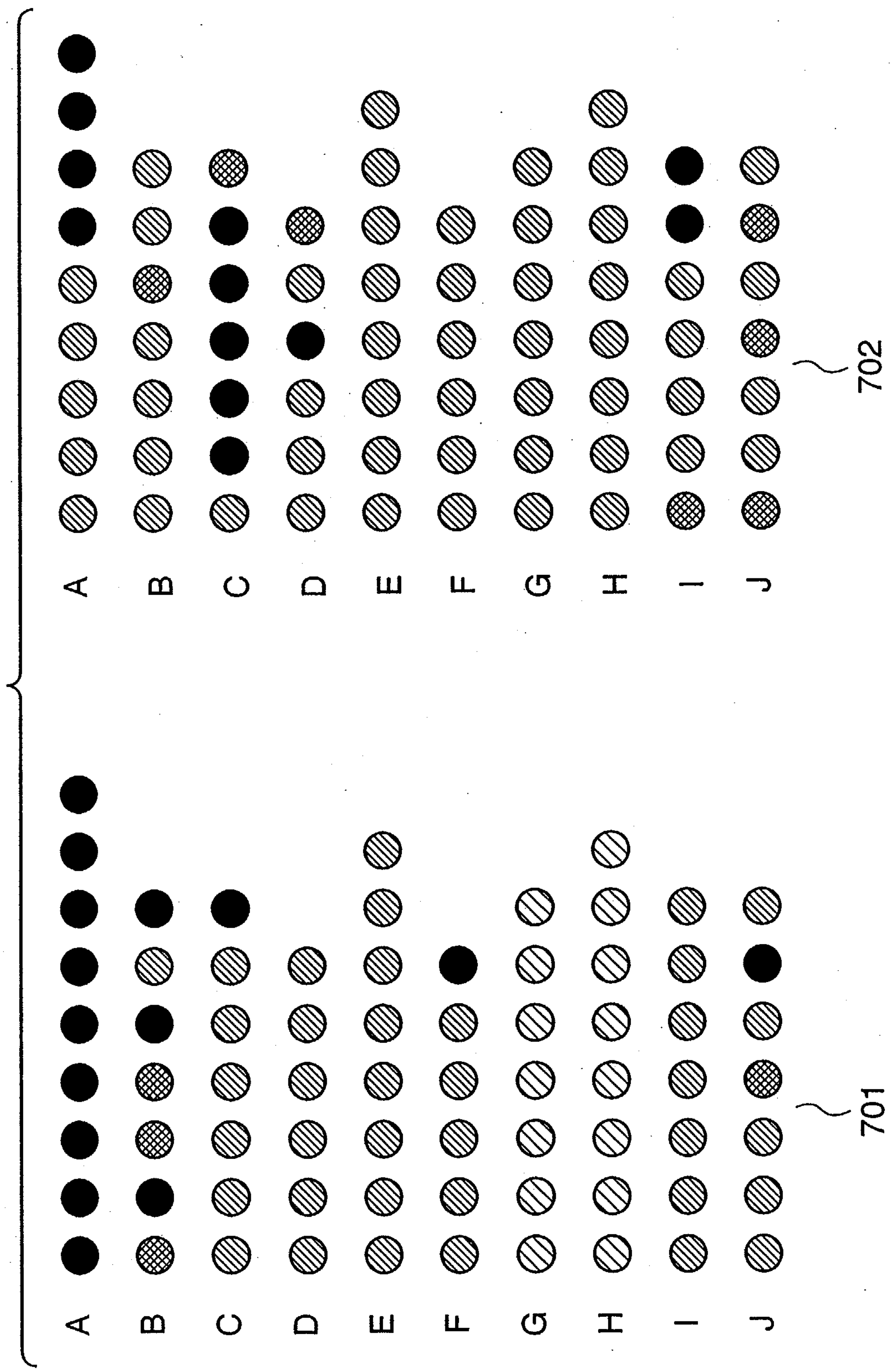

FIG. 8A

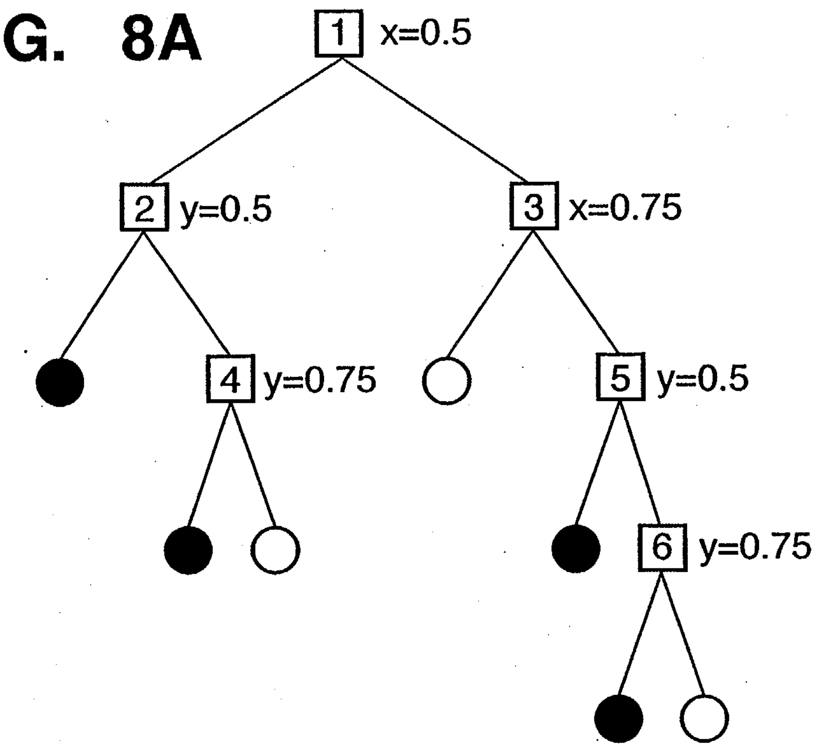
1 x=0.5
2 y=0.5
3 x=0.75
4 y=0.75
5 y=0.5
6 y=0.75

FIG. 8B

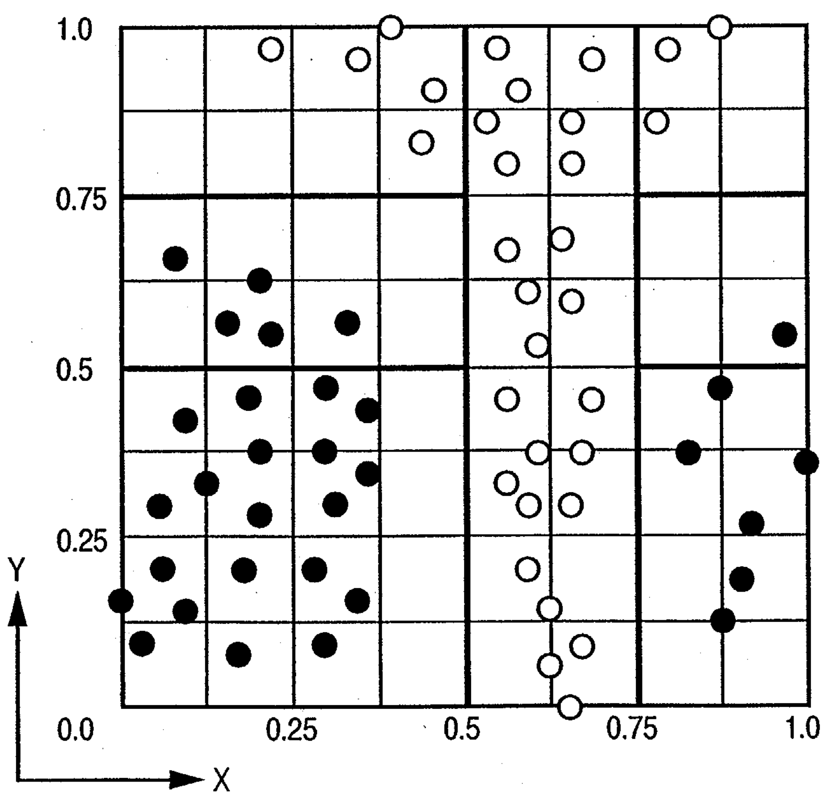
1.0
0.75
0.5
0.25
0.0
0.25
0.5
0.75
1.0
Y
X

FIG. 9

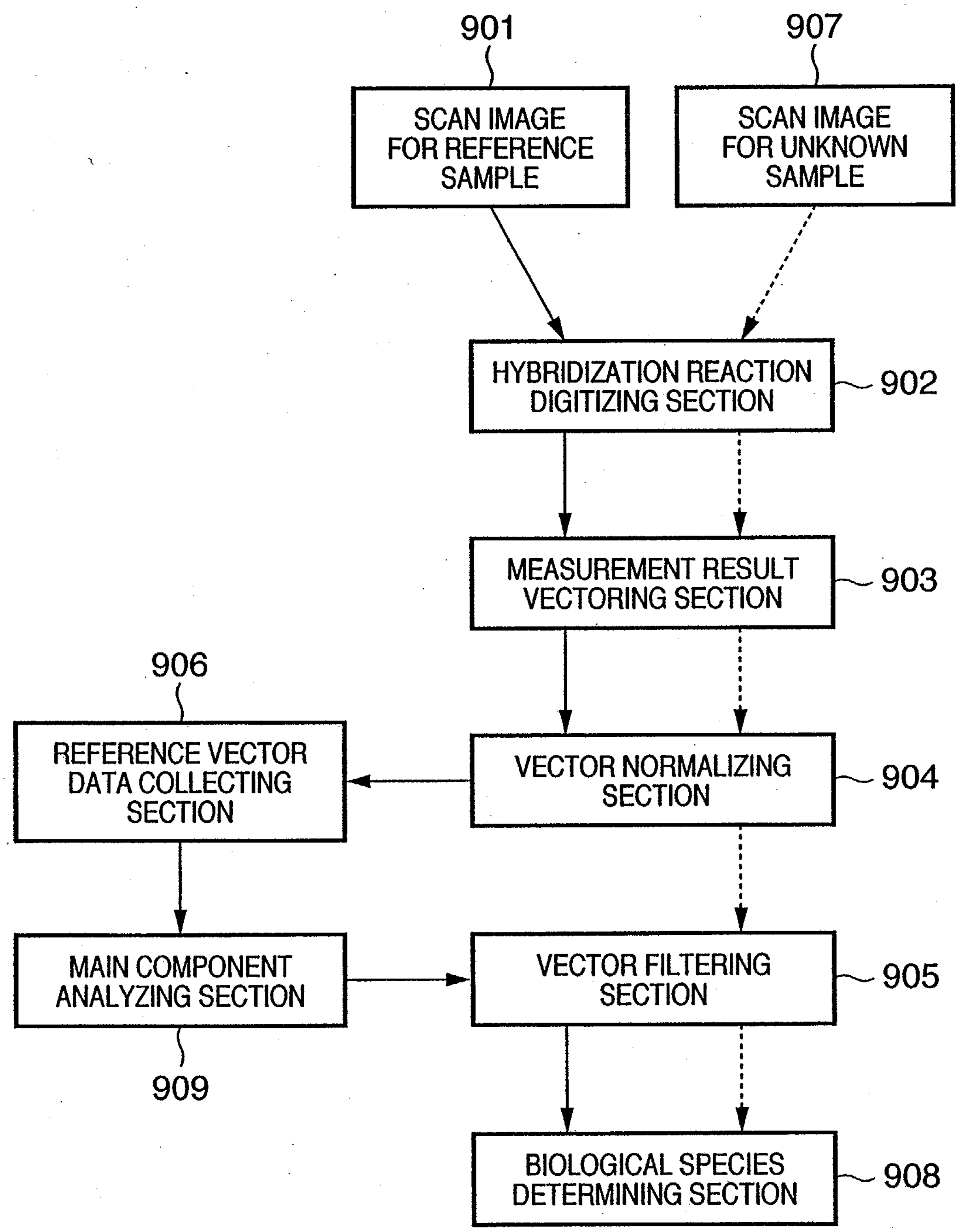
901
SCAN IMAGE FOR REFERENCE SAMPLE
907
SCAN IMAGE FOR UNKNOWN SAMPLE
HYBRIDIZATION REACTION DIGITIZING SECTION
902
MEASUREMENT RESULT VECTORING SECTION
903
906
REFERENCE VECTOR DATA COLLECTING SECTION
VECTOR NORMALIZING SECTION
904
MAIN COMPONENT ANALYZING SECTION
VECTOR FILTERING SECTION
905
909
BIOLOGICAL SPECIES DETERMINING SECTION
908

FIG. 10

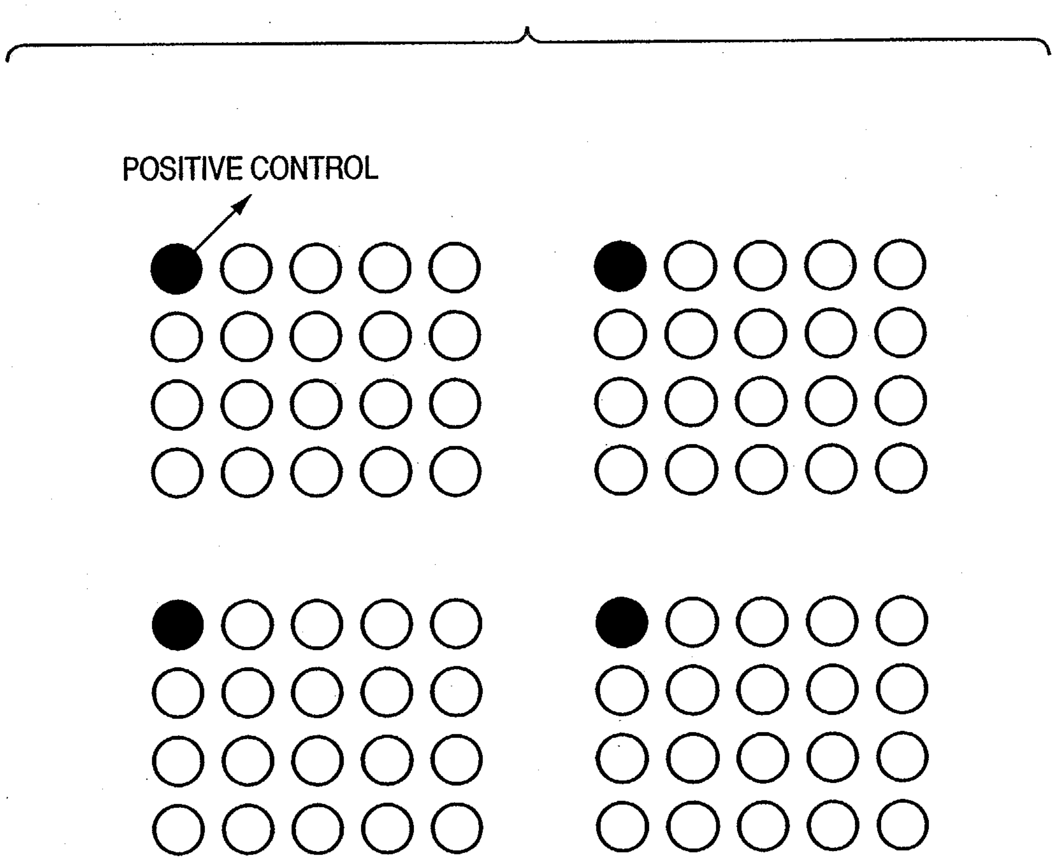

FIG. 11

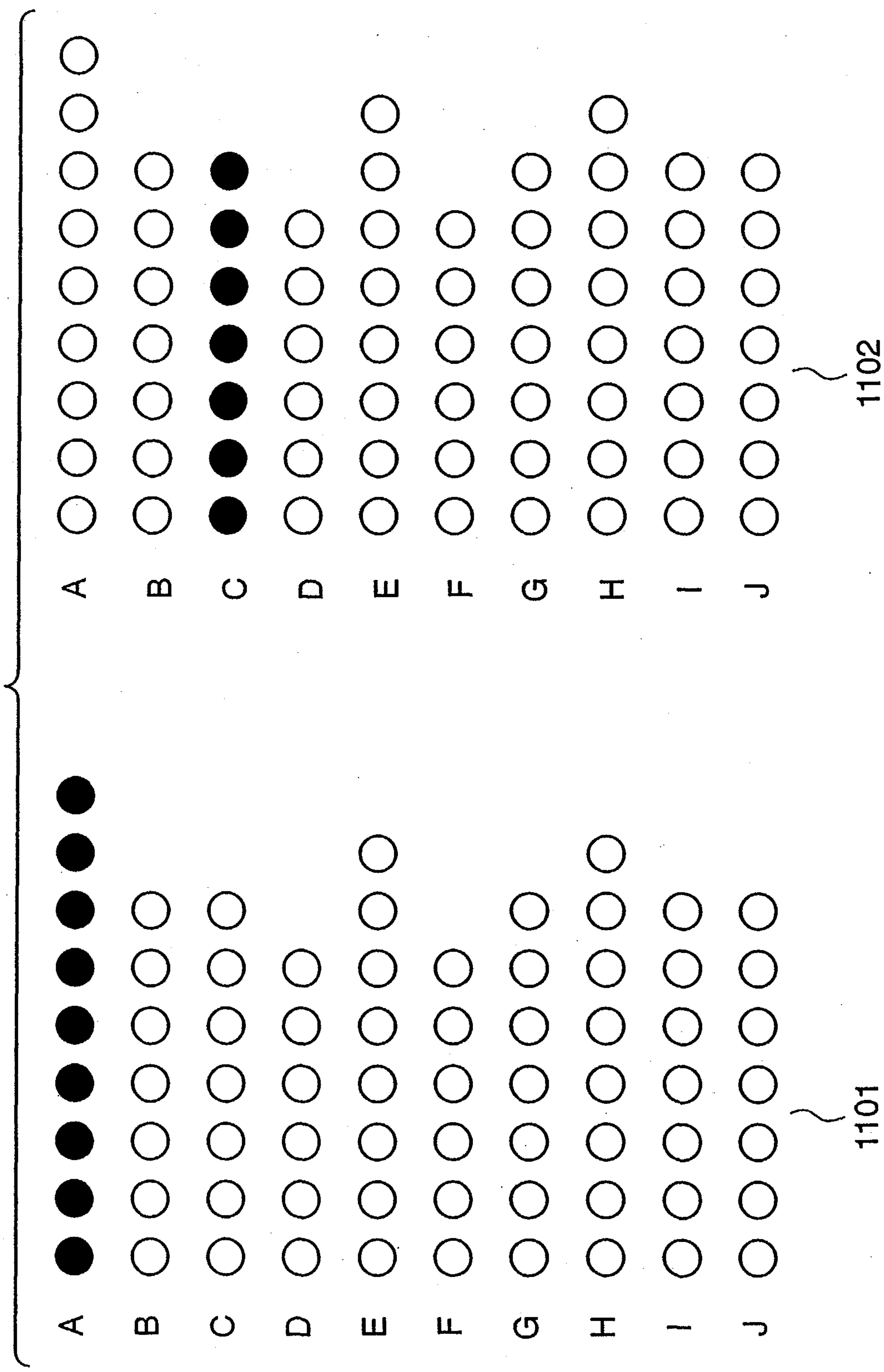

FIG. 12

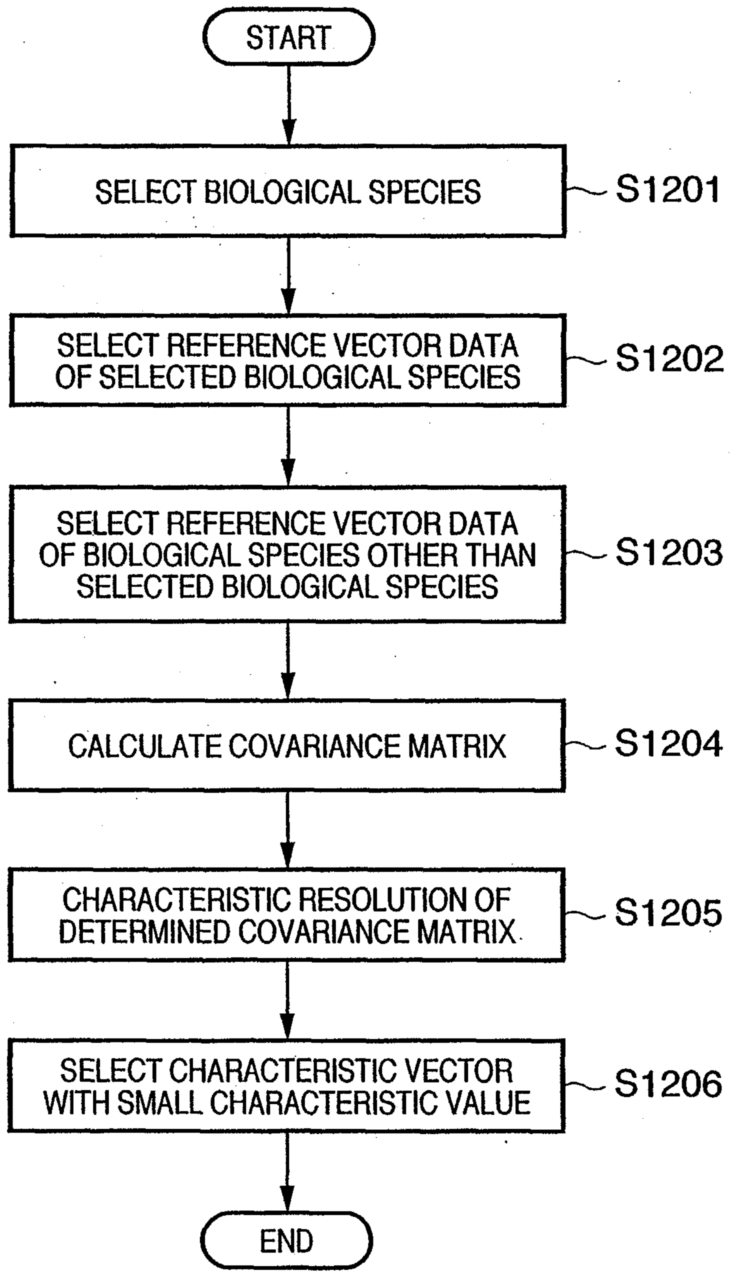
START
SELECT BIOLOGICAL SPECIES
S1201
SELECT REFERENCE VECTOR DATA OF SELECTED BIOLOGICAL SPECIES
S1202
SELECT REFERENCE VECTOR DATA OF BIOLOGICAL SPECIES OTHER THAN SELECTED BIOLOGICAL SPECIES
S1203
CALCULATE COVARIANCE MATRIX
S1204
CHARACTERISTIC RESOLUTION OF DETERMINED COVARIANCE MATRIX
S1205
SELECT CHARACTERISTIC VECTOR WITH SMALL CHARACTERISTIC VALUE
S1206
END

FIG. 13

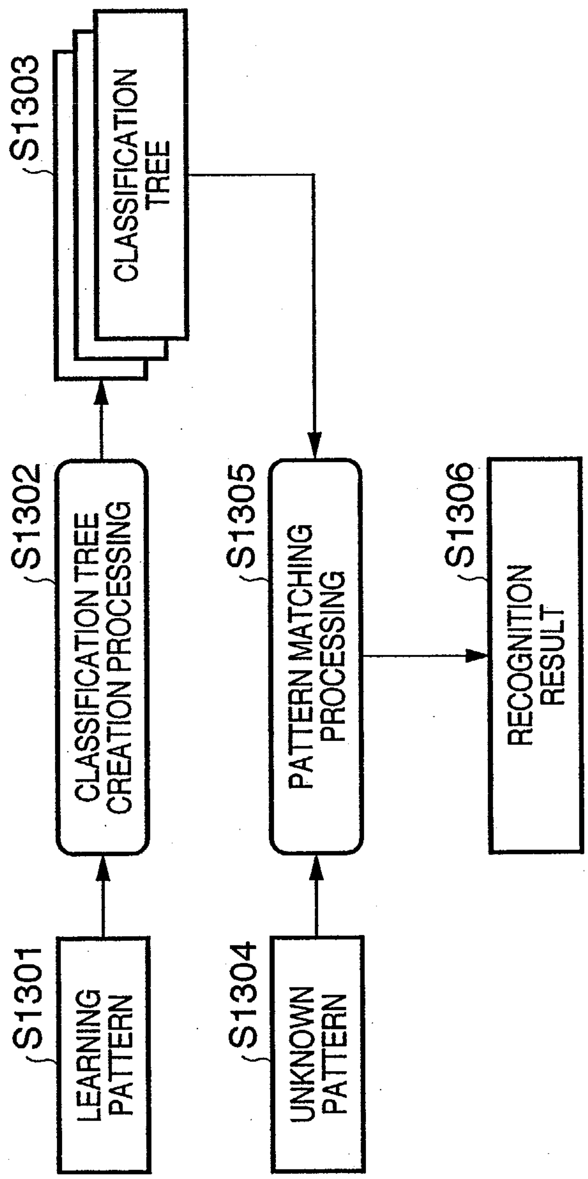
S1301
LEARNING PATTERN
S1302
CLASSIFICATION TREE CREATION PROCESSING
S1303
CLASSIFICATION TREE
S1304
UNKNOWN PATTERN
S1305
PATTERN MATCHING PROCESSING
S1306
RECOGNITION RESULT

FIG. 14

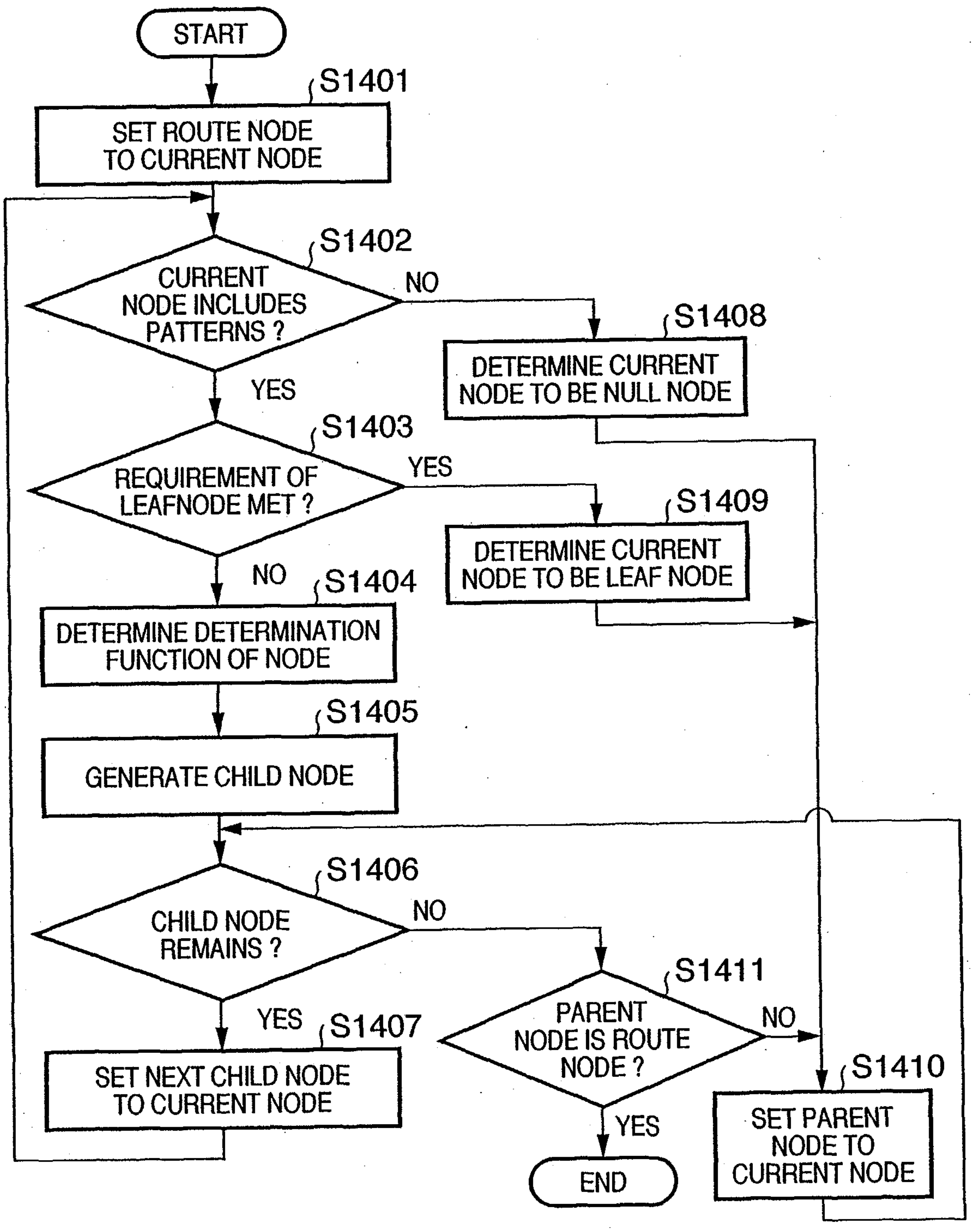
START
S1401
SET ROUTE NODE TO CURRENT NODE
S1402
CURRENT NODE INCLUDES PATTERNS ?
NO
S1408
DETERMINE CURRENT NODE TO BE NULL NODE
YES
S1403
REQUIREMENT OF LEAFNODE MET ?
YES
S1409
DETERMINE CURRENT NODE TO BE LEAF NODE
NO
S1404
DETERMINE DETERMINATION FUNCTION OF NODE
S1405
GENERATE CHILD NODE
S1406
CHILD NODE REMAINS ?
NO
S1411
PARENT NODE IS ROUTE NODE ?
NO
YES
S1407
SET NEXT CHILD NODE TO CURRENT NODE
YES
END
S1410
SET PARENT NODE TO CURRENT NODE

FIG. 15

SEQUENCE LISTING

<110> CANON KABUSHIKIKAISHA

<120> INFORMATION PROCESSING APPARATUS, INFORMATION PROCESSING METHOD, STORAGE MEDIUM AND PROGRAM

<130> XXXXX

<150> 2003-140793
<151> 2003-05-19

<160> 78

<170> PatentIn version 3.2

<210> 1
<211> 22
<212> DNA
<213> Artificial

<220>
<223> Synthesized DNA probe

<400> 1
cagagagctt gctctcgggt ga 22

<210> 2
<211> 26
<212> DNA
<213> Artificial

<220>
<223> Synthesized DNA probe

<400> 2
gggaggaagg tgttgtggtt aataac 26

<210> 3
<211> 26
<212> DNA
<213> Artificial

<220>
<223> Synthesized DNA probe

<400> 3
ggtgttgtgg ttaataacca cagcaa 26

<210> 4
<211> 22
<212> DNA
<213> Artificial

<220>
<223> Synthesized DNA probe

<400> 4
gcggtctgtc aagtcggatg tg 22

<210> 5
<211> 25
<212> DNA
<213> Artificial

<220>
<223> Synthesized DNA probe

<400> 5
attcgaaact ggcaggctag agtct 25

<210> 6
<211> 25
<212> DNA
<213> Artificial

<220>
<223> Synthesized DNA probe

<400> 6
taaccacagc aattgacgtt acccg 25

<210> 7
<211> 24
<212> DNA
<213> Artificial

<220>
<223> Synthesized DNA probe

<400> 7
gcaattgacg ttacccgcag aaga 24

<210> 8
<211> 26
<212> DNA
<213> Artificial

<220>
<223> Synthesized DNA probe

<400> 8
gaaccgcatg gttcaaaagt gaaaga 26

<210> 9
<211> 24
<212> DNA
<213> Artificial

<220>
<223> Synthesized DNA probe

<400> 9
cacttataga tggatccgcg ctgc 24

<210> 10
<211> 26
<212> DNA
<213> Artificial

<220>
<223> Synthesized DNA probe

<400> 10
tgcacatctt gacggtacct aatcag 26

<210> 11
<211> 22
<212> DNA
<213> Artificial

<220>
<223> Synthesized DNA probe

<400> 11
ccccttagtg ctgcagctaa cg 22

<210> 12
<211> 23
<212> DNA

<213> Artificial

<220>
<223> Synthesized DNA probe

<400> 12
aatacaaagg gcagcgaaac cgc 23

<210> 13
<211> 25
<212> DNA
<213> Artificial

<220>
<223> Synthesized DNA probe

<400> 13
ccggtggagt aaccttttag gagct 25

<210> 14
<211> 25
<212> DNA
<213> Artificial

<220>
<223> Synthesized DNA probe

<400> 14
taacctttta ggagctagcc gtcga 25

<210> 15
<211> 23
<212> DNA
<213> Artificial

<220>
<223> Synthesized DNA probe

<400> 15
tttaggagct agccgtcgaa ggt 23

<210> 16
<211> 23
<212> DNA
<213> Artificial

<220>
<223> Synthesized DNA probe

<400> 16
tagccgtcga aggtgggaca aat 23

<210> 17
<211> 22
<212> DNA
<213> Artificial

<220>
<223> Synthesized DNA probe

<400> 17
gaacagacga ggagcttgct cc 22

<210> 18
<211> 26
<212> DNA
<213> Artificial

<220>

<223> Synthesized DNA probe

<400> 18
tagtgaaaga cggttttgct gtcact 26

<210> 19
<211> 26
<212> DNA
<213> Artificial

<220>
<223> Synthesized DNA probe

<400> 19
taagtaacta tgcacgtctt gacggt 26

<210> 20
<211> 26
<212> DNA
<213> Artificial

<220>
<223> Synthesized DNA probe

<400> 20
gacccctcta gagatagagt tttccc 26

<210> 21
<211> 24
<212> DNA
<213> Artificial

<220>
<223> Synthesized DNA probe

<400> 21
agtaaccatt tggagctagc cgtc 24

<210> 22
<211> 23
<212> DNA
<213> Artificial

<220>
<223> Synthesized DNA probe

<400> 22
gagcttgctc ctctgacgtt agc 23

<210> 23
<211> 22
<212> DNA
<213> Artificial

<220>
<223> Synthesized DNA probe

<400> 23
agccggtgga gtaaccattt gg 22

<210> 24
<211> 22
<212> DNA
<213> Artificial

<220>
<223> Synthesized DNA probe

<400> 24

ctcttgccat cggatgtgcc ca 22

<210> 25
<211> 26
<212> DNA
<213> Artificial

<220>
<223> Synthesized DNA probe

<400> 25
ataccttttgc tcattgacgt tacccg 26

<210> 26
<211> 24
<212> DNA
<213> Artificial

<220>
<223> Synthesized DNA probe

<400> 26
tttgctcatt gacgttaccc gcag 24

<210> 27
<211> 24
<212> DNA
<213> Artificial

<220>
<223> Synthesized DNA probe

<400> 27
actggcaagc ttgagtctcg taga 24

<210> 28
<211> 23
<212> DNA
<213> Artificial

<220>
<223> Synthesized DNA probe

<400> 28
atacaaagag aagcgacctc gcg 23

<210> 29
<211> 25
<212> DNA
<213> Artificial

<220>
<223> Synthesized DNA probe

<400> 29
cggacctcat aaagtgcgtc gtagt 25

<210> 30
<211> 26
<212> DNA
<213> Artificial

<220>
<223> Synthesized DNA probe

<400> 30
gcggggagga agggagtaaa gttaat 26

<210> 31
<211> 23
<212> DNA
<213> Artificial

<220>
<223> Synthesized DNA probe

<400> 31
tagcacagag agcttgctct cgg 23

<210> 32
<211> 23
<212> DNA
<213> Artificial

<220>
<223> Synthesized DNA probe

<400> 32
tcatgccatc agatgtgccc aga 23

<210> 33
<211> 25
<212> DNA
<213> Artificial

<220>
<223> Synthesized DNA probe

<400> 33
cggggaggaa ggcgataagg ttaat 25

<210> 34
<211> 25
<212> DNA
<213> Artificial

<220>
<223> Synthesized DNA probe

<400> 34
ttcgattgac gttacccgca gaaga 25

<210> 35
<211> 26
<212> DNA
<213> Artificial

<220>
<223> Synthesized DNA probe

<400> 35
ggtctgtcaa gtcggatgtg aaatcc 26

<210> 36
<211> 24
<212> DNA
<213> Artificial

<220>
<223> Synthesized DNA probe

<400> 36
gcaggctaga gtcttgtaga gggg 24

<210> 37
<211> 24
<212> DNA

<213> Artificial

<220>
<223> Synthesized DNA probe

<400> 37
tgagggagaa agtgggggat cttc 24

<210> 38
<211> 25
<212> DNA
<213> Artificial

<220>
<223> Synthesized DNA probe

<400> 38
tcagatgagc ctaggtcgga ttagc 25

<210> 39
<211> 24
<212> DNA
<213> Artificial

<220>
<223> Synthesized DNA probe

<400> 39
gagctagagt acggtagagg gtgg 24

<210> 40
<211> 25
<212> DNA
<213> Artificial

<220>
<223> Synthesized DNA probe

<400> 40
gtacggtaga gggtggtgga atttc 25

<210> 41
<211> 24
<212> DNA
<213> Artificial

<220>
<223> Synthesized DNA probe

<400> 41
gaccacctgg actgatactg acac 24

<210> 42
<211> 25
<212> DNA
<213> Artificial

<220>
<223> Synthesized DNA probe

<400> 42
tggccttgac atgctgagaa ctttc 25

<210> 43
<211> 23
<212> DNA
<213> Artificial

<220>

<223> Synthesized DNA probe

<400> 43
ttagttacca gcacctcggg tgg 23

<210> 44
<211> 22
<212> DNA
<213> Artificial

<220>
<223> Synthesized DNA probe

<400> 44
tagtctaacc gcaaggggga cg 22

<210> 45
<211> 22
<212> DNA
<213> Artificial

<220>
<223> Synthesized DNA probe

<400> 45
tagcacaggg agcttgctcc ct 22

<210> 46
<211> 25
<212> DNA
<213> Artificial

<220>
<223> Synthesized DNA probe

<400> 46
aggtggtgag cttaatacgc tcatc 25

<210> 47
<211> 26
<212> DNA
<213> Artificial

<220>
<223> Synthesized DNA probe

<400> 47
tcatcaattg acgttactcg cagaag 26

<210> 48
<211> 26
<212> DNA
<213> Artificial

<220>
<223> Synthesized DNA probe

<400> 48
actgcatttg aaactggcaa gctaga 26

<210> 49
<211> 24
<212> DNA
<213> Artificial

<220>
<223> Synthesized DNA probe

<400> 49

ttatcctttg ttgcagcttc ggcc 24

<210> 50
<211> 23
<212> DNA
<213> Artificial

<220>
<223> Synthesized DNA probe

<400> 50
actttcagcg aggaggaagg tgg 23

<210> 51
<211> 25
<212> DNA
<213> Artificial

<220>
<223> Synthesized DNA probe

<400> 51
agtagaacgc tgaaggagga gcttg 25

<210> 52
<211> 25
<212> DNA
<213> Artificial

<220>
<223> Synthesized DNA probe

<400> 52
cttgcatcac taccagatgg acctg 25

<210> 53
<211> 26
<212> DNA
<213> Artificial

<220>
<223> Synthesized DNA probe

<400> 53
tgagagtgga aagttcacac tgtgac 26

<210> 54
<211> 26
<212> DNA
<213> Artificial

<220>
<223> Synthesized DNA probe

<400> 54
gctgtggctt aaccatagta ggcttt 26

<210> 55
<211> 23
<212> DNA
<213> Artificial

<220>
<223> Synthesized DNA probe

<400> 55
aagcggctct ctggcttgta act 23

<210> 56
<211> 24
<212> DNA
<213> Artificial

<220>
<223> Synthesized DNA probe

<400> 56
tagacccttt ccggggttta gtgc 24

<210> 57
<211> 26
<212> DNA
<213> Artificial

<220>
<223> Synthesized DNA probe

<400> 57
gacggcaagc taatctctta aagcca 26

<210> 58
<211> 24
<212> DNA
<213> Artificial

<220>
<223> Synthesized DNA probe

<400> 58
gcttgggaat ctggcttatg gagg 24

<210> 59
<211> 23
<212> DNA
<213> Artificial

<220>
<223> Synthesized DNA probe

<400> 59
tgccatagga tgagcccaag tgg 23

<210> 60
<211> 26
<212> DNA
<213> Artificial

<220>
<223> Synthesized DNA probe

<400> 60
cttgggaatg tactgacgct catgtg 26

<210> 61
<211> 23
<212> DNA
<213> Artificial

<220>
<223> Synthesized DNA probe

<400> 61
ggattgggct tagagcttgg tgc 23

<210> 62
<211> 22
<212> DNA

<213> Artificial

<220>
<223> Synthesized DNA probe

<400> 62
tacagaggga agcgaagctg cg 22

<210> 63
<211> 26
<212> DNA
<213> Artificial

<220>
<223> Synthesized DNA probe

<400> 63
ggcgtttacc acggtatgat tcatga 26

<210> 64
<211> 23
<212> DNA
<213> Artificial

<220>
<223> Synthesized DNA probe

<400> 64
aatgcctacc aagcctgcga tct 23

<210> 65
<211> 25
<212> DNA
<213> Artificial

<220>
<223> Synthesized DNA probe

<400> 65
tatcggaaga tgaaagtgcg ggact 25

<210> 66
<211> 23
<212> DNA
<213> Artificial

<220>
<223> Synthesized DNA probe

<400> 66
ttctttcctc ccgagtgctt gca 23

<210> 67
<211> 25
<212> DNA
<213> Artificial

<220>
<223> Synthesized DNA probe

<400> 67
aacacgtggg taacctaccc atcag 25

<210> 68
<211> 24
<212> DNA
<213> Artificial

<220>

<223> Synthesized DNA probe

<400> 68
atggcataag agtgaaaggc gctt 24

<210> 69
<211> 22
<212> DNA
<213> Artificial

<220>
<223> Synthesized DNA probe

<400> 69
gacccgcggt gcattagcta gt 22

<210> 70
<211> 25
<212> DNA
<213> Artificial

<220>
<223> Synthesized DNA probe

<400> 70
ggacgttagt aactgaacgt cccct 25

<210> 71
<211> 22
<212> DNA
<213> Artificial

<220>
<223> Synthesized DNA probe

<400> 71
ctcaaccggg gagggtcatt gg 22

<210> 72
<211> 22
<212> DNA
<213> Artificial

<220>
<223> Synthesized DNA probe

<400> 72
ttggagggtt tccgcccttc ag 22

<210> 73
<211> 23
<212> DNA
<213> Artificial

<220>
<223> Synthesized DNA for forward primer

<400> 73
gcggcgtgcc taatacatgc aag 23

<210> 74
<211> 23
<212> DNA
<213> Artificial

<220>
<223> Synthesized DNA for forward primer

<400> 74

gcggcaggcc taacacatgc aag 23

<210> 75
<211> 23
<212> DNA
<213> Artificial

<220>
<223> Synthesized DNA for forward primer

<400> 75
gcggcaggct taacacatgc aag 23

<210> 76
<211> 22
<212> DNA
<213> Artificial

<220>
<223> Synthesized DNA for reverse primer

<400> 76
atccagccgc accttccgat ac 22

<210> 77
<211> 22
<212> DNA
<213> Artificial

<220>
<223> Synthesized DNA for reverse primer

<400> 77
atccaaccgc aggttcccct ac 22

<210> 78
<211> 22
<212> DNA
<213> Artificial

<220>
<223> Synthesized DNA for reverse primer

<400> 78
atccagccgc aggttcccct ac 22